# EUROPÄISCHE PATENTANMELDUNG

(11) **EP 1 669 369 A2**
(43) Veröffentlichungstag der Anmeldung: **14.06.2006**
(21) Anmeldenummer: 05026934.9
(22) Anmeldetag: 31.10.2002
(51) Int. Cl.: C07K 14/34, C12P 13/08, C12Q 1/68, C12N 15/63, C12N 15/77, C12N 1/21, C12P 1/04, C12R 1/15

(54) **Nukleinsäuresequenz die für das OPCA Gen kodiert**

(30) Priorität: 05.11.2001 DE 10154180
(62) Teilanmeldung aus: 02796537.5
(71) Anmelder: BASF Aktiengesellschaft, 67056 Ludwigshafen (DE)
(72) Erfinder: Zelder, Oskar, Dr., 67346 Speyer (DE); Pompejus, Markus, Dr., Seoul 140-210 (KR); Schröder, Hartwig, Dr., 69226 Nussloch (DE); Kröger, Burkhard, Dr., 67117 Limburgerhof (DE); Klopprogge, Corinna, Dr., 68239 Mannheim (DE); Haberhauer, Gregor, 67117 Limburgerhof (DE)

(57) **Zusammenfassung**

Die Erfindung betrifft neue Nukleinsäuremoleküle, deren Verwendung zur Konstruktion von gentechnisch verbesserten Mikroorganismen und Verfahren zur Herstellung von Feinchemikalien, insbesondere Aminosäuren mit Hilfe dieser.gentechnisch verbesserten Mikroorganismen.

## Beschreibung

### Hintergrund der Erfindung

Bestimmte Produkte und Nebenprodukte von natürlich vorkommenden Stoffwechselprozessen in Zellen werden in vielen Industriezweigen verwendet, einschließlich der Nahrungsmittel-, Futtermittel- , Kosmetik- und pharmazeutischen Industrie. Diese Moleküle, die gemeinsam als "Feinchemikalien" bezeichnet werden, umfassen organische Säuren, sowohl proteinogene als auch nicht-proteinogene Aminosäuren, Nukleotide und Nukleoside, Lipide und Fettsäuren, Diole, Kohlehydrate, aromatische Verbindungen, Vitamine und Cofaktoren sowie Enzyme. Ihre Produktion erfolgt am besten mittels Anzucht von Bakterien im Großmaßstab, die entwickelt wurden, um große Mengen des jeweils gewünschten Moleküls zu produzieren und sezernieren. Ein für diesen Zweck besonders geeigneter Organismus ist *Corynebacterium glutamicum,* ein gram-positives, nichtpathogenes Bakterium. Über Stammselektion ist eine Reihe von Mutantenstämmen entwickelt worden, die ein Sortiment wünschenswerter Verbindungen produzieren. Die Auswahl von Stämmen, die hinsichtlich der Produktion eines bestimmten Moleküls verbessert sind, ist jedoch ein zeitaufwendiges und schwieriges Verfahren.

### Zusammenfassung der Erfindung

Diese Erfindung stellt neuartige Nukleinsäuremoleküle bereit, die sich zur Identifizierung oder Klassifizierung von *Corynebacterium glutamicum* oder verwandten Bakterienarten verwenden lassen. *C. glutamicum* ist ein gram-positives, aerobes Bakterium, das gewöhnlich in der Industrie für die Produktion im Großmaßstab einer Reihe von Feinchemikalien und auch zum Abbau von Kohlenwasserstoffen (bspw. beim Überlaufen von Rohöl) und zur Oxidation von Terpenoiden gemeinhin verwendet wird. Die Nukleinsäuremoleküle können daher zum Identifizieren von Mikroorganismen eingesetzt werden, die sich zur Produktion von Feinchemikalien, bspw. durch Fermentationsverfahren, verwenden lassen. C. *glutamicum* selbst ist zwar nicht-pathogen, jedoch ist es mit anderen Corynebacterium-Arten, wie *Corynebacterium diphtheriae* (dem Erreger der Diphtherie) verwandt, die bedeutende Pathogene beim Menschen sind. Die Fähigkeit, das Vorhandensein von Corynebacterium-Arten zu identifizieren, kann daher auch eine signifikante klinische Bedeutung haben, z.B. bei diagnostischen Anwendungen. Diese Nukleinsäuremoleküle können zudem als Bezugspunkte zur Kartierung des *C. glutamicum*-Genoms oder von Genomen verwandter Organismen dienen.

Diese neuen Nukleinsäuremoleküle codieren Proteine, die hier als Genstabilitäts-, Genexpressions- oder Proteinsekretions-/Faltungs- (SES-) Proteine bezeichnet werden. Diese SES-Proteine können bspw. eine an der Reparatur oder Rekombination von DNA, Transposition von genetischem Material, Expression von Genen (d. h. die an der Transkription oder Translation beteiligt sind), Proteinfaltung oder Proteinsekretion in C. *glutamicum* beteiligte Funktion ausüben. Aufgrund der Verfügbarkeit von in *Corynebacterium glutamicum* verwendbaren Klonierungsvektoren, wie bspw. offenbart in Sinskey et al., US-Patent Nr. 4 649 119, und von Techniken zur genetischen Manipulation von C. *glutamicum* und den verwandten Brevibacterium-Arten (z.B. lactofermentum) (Yoshihama et al., J. Bacteriol. 162:591-597 (1985); Katsumata et al., J. Bacteriol. 159:306-311 (1984); und Santamaria et al. J. Gen. Microbiol. 130:2237-2246 (1984)), lassen sich die erfindungsgemäßen Nukleinsäuremoleküle zur genetischen Manipulation dieses Organismus verwenden, damit er ein effizienterer Produzent einer oder mehrerer Feinchemikalien wird. Diese verbesserte Produktion oder Effizienz der Produktion einer Feinchemikalie kann aufgrund einer direkten Auswirkung der Manipulation eines erfindungsgemäßen Gens oder aufgrund einer indirekten Auswirkung einer solchen Manipulation erfolgen.

Es gibt eine Reihe von Mechanismen, durch die die Veränderung eines erfindungsgemäßen SES-Proteins die Ausbeute, Produktion und/oder Effizienz der Produktion einer Feinchemikalie aus einem *C. glutamicum*-Stamm, der dieses veränderte Protein enthält, direkt beeinflussen kann. Zum Beispiel sollte die Modulation von Proteinen, die direkt an der Transkription oder Translation beteiligt sind (z.B. Polymerasen oder Ribosomen), so daß ihre Anzahl oder Aktivität gesteigert wird, die zelluläre Transkription oder Translation (oder die Geschwindigkeiten dieser Prozesse) insgesamt steigern. Diese erhöhte zelluläre Genexpression sollte solche Proteine umfassen, die an der Feinchemikalienbiosynthese beteiligt sind, so daß eine Steigerung der Ausbeute, Produktion oder Effizienz der Produktion einer oder mehrerer gewünschten Verbindungen erfolgen kann. Modifikationen der Transkriptions-/Translations-Proteinmaschinerie von *C. glutamicum,* so daß die Regulation dieser Proteine verändert wird, kann auch die erhöhte Expression von Genen, die an der Produktion von Feinchemikalien beteiligt sind, ermöglichen. Die Modulation der Aktivität einer Reihe von Proteinen, die an der Peptidfaltung beteiligt sind, kann eine Erhöhung der Gesamtproduktion korrekt gefalteter Moleküle in der Zelle ermöglichen, wodurch die Möglichkeit erhöht wird, daß gewünschte Proteine (z.B. Feinchemikalienbiosynthese-Proteine) richtig funktionieren. Ferner kann es durch Mutation von an der Sekretion aus *C. glutamicum* beteiligten Proteinen, so daß ihre Anzahl oder Aktivität erhöht ist, möglich sein, die Sekretion einer Feinchemikalie (z.B. eines Enzyms) aus Zellen in der Fermentationskultur zu erhöhen, aus der sie leicht gewonnen werden kann.

Die genetische Modifikation der erfindungsgemäßen SES-Moleküle kann auch zu einer indirekten Modulation der Produktion einer oder mehrerer Feinchemikalien führen. Beispielsweise kann man durch Erhöhen der Anzahl oder Aktivität eines erfindungsgemäßen DNA-Reparatur- oder -Rekombinationsproteins die Fähigkeit der Zelle, eine DNA-Schädigung zu entdecken und zu reparieren, erhöhen. Dies sollte die Fähigkeit der Zelle, ein mutiertes Gen in ihrem Genom zu halten, wirksam erhöhen und dadurch die Wahrscheinlichkeit erhöhen, daß ein gentechnologisch in C. *glutami*cum eingebrachtes Transgen (das z.B. ein Protein codiert, das die Biosynthese einer Feinchemikalie steigert) nicht während der Züchtung des Mikroorganismus verloren geht. Dagegen kann es durch Verringern der Anzahl oder Aktivität eines oder mehrerer DNA-Reparatur- oder -Rekombinationsproteine möglich sein, die genetische Instabilität des Organismus zu steigern. Diese Manipulationen sollten die Fähigkeit des Organismus, durch Mutagenese modifiziert zu werden, verbessern, ohne daß die eingebrachte Mutation berichtigt wird. Das gleiche gilt für Proteine, die an der Transposition oder Umlagerung genetischer Elemente in C. *glutamicum* beteiligt sind (z.B. Transposons). Durch Mutagenese dieser Proteine, so daß ihre Anzahl oder Aktivität entweder gesteigert oder verringert wird, ist es möglich, gleichzeitig die genetische Stabilität des Mikroorganismus zu steigern oder zu verringern. Dies hat eine bedeutende Auswirkung darauf, daß eine andere Mutation in *C. glutamicum* eingebracht und die eingebrachte Mutation beibehalten werden kann. Transposons bieten ebenfalls einen geeigneten Mechanismus, durch den die Mutagenese von *C. glutamicum* durchgeführt werden kann; die Duplikation gewünschter Gene (z.B. von Feinchemikalienbiosynthese-Genen) läßt sich leicht mittels Transposonmutagenese durchführen, wie auch die Disruption ungewünschter Gene (z.B. Gene, die am Abbau gewünschter Feinchemikalien beteiligt sind).

Durch die Modulation eines oder mehrerer Proteine (z.B. Sigma-Faktoren), die an der Regulation der Transkription oder Translation in Reaktion auf besondere Umweltbedingungen beteiligt sind, kann es möglich sein, die Zelle daran zu hindern, daß sie die Proteinsynthese unter ungünstigen Umweltbedingungen, wie man sie in einer Fermenterkultur im Großmaßstab antrifft, verlangsamt oder beendet. Dies sollte zu erhöhter Genexpression führen, was wiederum die gesteigerte Biosynthese gewünschter Feinchemikalien unter diesen Bedingungen ermöglichen kann. Die Mutagenese von an Sekretionssystemen beteiligten Proteinen kann zu modulierten Sekretionsraten führen. Viele dieser sezernierten Proteine haben Funktionen, die für die Zellebensfähigkeit wichtig sind (z.B. Zelloberflächenproteasen oder -Rezeptoren). Eine Änderung des Sekretionswegs, so daß diese Proteine leichter an ihren extrazellulären Ort transportiert werden, kann die Gesamtlebensfähigkeit der Zelle erhöhen und somit zu höheren Zahlen an C. *gluta*micum-Zellen führen, die Feinchemikalien während eine Züchtung im Großmaßstab produzieren können. Ferner ist der Sekretionsapparat (z.B. das sec-System) bekanntlich auch an der Insertion von integralen Membranproteinen (z.B. Poren, Kanälen oder Transportern) in die Membran beteiligt. So kann die Modulation der Aktivität von Proteinen, die an der Proteinsekretion aus C. *glutamicum* beteiligt sind, die Fähigkeit der Zelle zur Ausscheidung von Abfallprodukten oder zum Import notwendiger Metabolite beeinflussen. Ist die Aktivität dieser sekretorischen Proteine erhöht, kann ebenfalls die Fähgikeit der Zelle zur Produktion von Feinchemikalien erhöht sein. Ist die Aktivität dieser sekretorischen Proteine verringert, können nicht genügend Nährstoffe zur Unterstützung der Überproduktion gewünschter Verbindungen vorhanden sein, oder Abfallprodukte können diese Biosynthese stören.

Die Erfindung stellt neue Nukleinsäuremoleküle bereit, die Proteine codieren, die hier als SES-Proteine bezeichnet werden und bspw an der Reparatur oder Rekombination von DNA, Transposition von genetischem Material, Expression von Genen (d. h. den Transkriptions- oder Translationsprozessen), Proteinfaltung oder Proteinsekretion in *Corynebacterium glutamicum* beteiligt sein können. Nukleinsäuremoleküle, die ein SES-Protein codieren, werden hier als SES-Nukleinsäuremoleküle bezeichnet. Bei einer bevorzugten Ausführungsform ist ein SES-Protein an der Verbesserung oder Verringerung der genetischen Stabilität in C. *glutami*cum, der Expression von Genen (z.B. bei der Transkription oder Translation) oder der Proteinfaltung in diesem Organismus oder an der Proteinsekretion aus *C. glutamicum* beteiligt. Beispiele für solche Proteine sind diejenigen, die von den in Tabelle 1 angegebenen Genen codiert werden.

Ein Aspekt der Erfindung betrifft folglich isolierte Nukleinsäuremoleküle (bspw. cDNAs), umfassend eine Nukleotidsequenz, die ein SES-Protein oder biologisch aktive Abschnitte davon codiert, sowie Nukleinsäurefragmente, die sich als Primer oder Hybridisierungssonden zum Nachweis oder zur Amplifikation von SEScodierender Nukleinsäure (bspw. DNA oder mRNA) eignen. Bei besonders bevorzugten Ausführungsformen umfaßt das isolierte Nukleinsäuremolekül eine der in Anhang A aufgeführten Nukleotidsequenzen oder den codierenden Bereich einer dieser Nukleotidsequenzen oder ein Komplement davon. In anderen bevorzugten Ausführungsformen codiert das isolierte Nukleinsäuremolekül eine der in Anhang B aufgeführten Aminosäuresequenzen. Die bevorzugten erfindungsgemäßen SES-Proteine besitzen ebenfalls vorzugsweise mindestens eine der hier beschriebenen SES-Aktivitäten.

Als Anhang A werden im folgenden die Nukleinsäuresequenzen des Sequenzprotokolls zusammen mit den in Tabelle 1 beschriebenen Sequenzveränderungen an der jeweiligen Position definiert.

Als Anhang B werden im folgenden die Polypeptidsequenzen des Sequenzprotokolls zusammen mit den in Tabelle 1 beschriebenen Sequenzveränderungen an der jeweiligen Position definiert.

Bei einer weiteren Ausführungsform ist das isolierte Nukleinsäuremolekül mindestens 15 Nukleotide lang und hybridisiert unter stringenten Bedingungen an ein Nukleinsäuremolekül, das eine Nukleotidsequenz aus Anhang A umfaßt. Das isolierte Nukleinsäuremolekül entspricht vorzugsweise einem natürlich vorkommenden Nukleinsäuremolekül. Die isolierte Nukleinsäure codiert stärker bevorzugt ein natürlich vorkommendes *C. glutamicum*-SES-Protein oder einen biologisch aktiven Abschnitt davon.

Ein weiterer Aspekt der Erfindung betrifft Vektoren, bspw. rekombinante Expressionsvektoren, die die erfindungsgemäßen Nukleinsäuremoleküle enthalten, und Wirtszellen, in die diese Vektoren eingebracht worden sind. Bei einer Ausführungsform wird diese Wirtszelle zur Herstellung eines SES-Proteins verwendet, indem die Wirtszelle in einem geeigneten Medium gezüchtet wird. Das SES-Protein kann dann aus dem Medium oder der Wirtszelle isoliert werden.

Ein weiterer Aspekt der Erfindung betrifft einen genetisch veränderten Mikroorganismus, bei dem ein SES-Gen eingebracht oder verändert worden ist. Das Genom des Mikroorganismus ist bei einer Ausführungsform durch Einbringen mindestens eines erfindungsgemäßen Nukleinsäuremoleküls verändert worden, das die mutierte SES-Sequenz als Transgen codiert. Bei einer anderen Ausführungsform ist ein endogenes SES-Gen im Genom des Mikroorganismus durch homologe Rekombination mit einem veränderten SES-Gen verändert, z.B. funktionell disruptiert, worden. Der Mikroorganismus gehört bei einer bevorzugten Ausführungsform zur Gattung *Corynebacterium* oder *Brevibacterium*, wobei *Corynebacterium glutamicum* besonders bevorzugt ist. Der Mikroorganismus wird in einer bevorzugten Ausführungsform auch zur Herstellung einer gewünschten Verbindung, wie einer Aminosäure, verwendet, wobei Lysin besonders bevorzugt ist.

Ein weiterer Aspekt der Erfindung betrifft ein isoliertes SES-Protein oder einen Abschnitt, bspw. einen biologisch aktiven Abschnitt, davon. Das isolierte SES-Protein oder sein Abschnitt kann in einer bevorzugten Ausführungsform an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teilnehmen. Bei einer weiteren bevorzugten Ausführungsform ist das isolierte SES-Protein oder ein Abschnitt davon hinreichend homolog zu einer Aminosäuresequenz von Anhang B, so daß das Protein oder sein Abschnitt die Fähigkeit behält, bspw. an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teilzunehmen.

Eine weitere bevorzugte Ausführungsform sind Wirtszellen, die mehr als eine der in Anhang A beschriebenen Nukleinsäuremoleküle besitzen. Solche Wirtszellen lassen sich auf verschiedene dem Fachmann bekannte Wege herstellen. Beispielsweise können sie durch Vektoren, die mehrere der erfindungsgemäßen Nukleinsäuremoleküle tragen, transfiziert werden. Es ist aber auch möglich mit einem Vektor jeweils ein erfindungsgemäßes Nukleinsäuremolekül in die Wirtszelle einzubringen und deshalb mehrere Vektoren entweder gleichzeitig oder zeitlich abgestuft einzusetzen. Es können somit Wirtszellen konstruiert werden, die zahlreiche, bis zu mehreren Hundert der erfindungsgemäßen Nukleinsäuresequenzen tragen. Durch eine solche Akkumulation lassen sich häufig überadditive Effekte auf die Wirtszelle hinsichtlich der Feinchemikalien-Produktivität erzielen.

Die Erfindung stellt zudem ein isoliertes SES-Proteinpräparat bereit. Das SES-Protein umfaßt bei bevorzugten Ausführungsformen eine Aminosäuresequenz aus Anhang B. Bei einer weiteren bevorzugten Ausführungsform betrifft die Erfindung ein isoliertes Vollängenprotein, das zu einer vollständigen Aminosäuresequenz aus Anhang B (welche von einem offenen Leseraster in Anhang A codiert wird) im wesentlichen homolog ist.

Das SES-Polypeptid oder ein biologisch aktiver Abschnitt davon kann mit einem Nicht-SES-Polypeptid funktionsfähig verbunden werden, damit ein Fusionsprotein entsteht. Dieses Fusionsprotein hat bei bevorzugten Ausführungsformen eine andere Aktivität als das SES-Protein allein. Bei anderen bevorzugten Ausführungsformen nimmt dieses Fusionsprotein an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teil. Die Integration dieses Fusionsproteins in eine Wirtszelle moduliert bei besonders bevorzugten Ausführungsformen die Produktion einer gewünschten Verbindung von der Zelle.

Ein weiterer Aspekt der Erfindung betrifft ein Verfahren zur Herstellung einer Feinchemikalie. Das Verfahren sieht die Anzucht einer Zelle vor, die einen Vektor enthält, der die Expression eines erfindungsgemäßen SES-Nukleinsäuremoleküls bewirkt, so daß eine Feinchemikalie produziert wird. Dieses Verfahren umfaßt bei einer bevorzugten Ausführungsform zudem den Schritt der Gewinnung einer Zelle, die einen solchen Vektor enthält, wobei die Zelle mit einem Vektor transfiziert ist, der die Expression einer SES-Nukleinsäure bewirkt. Dieses Verfahren umfaßt bei einer weiteren bevorzugten Ausführungsform zudem den Schritt, bei dem die Feinchemikalie aus der Kultur gewonnen wird. Die Zelle gehört bei einer besonders bevorzugten Ausführungsform zur Gattung *Corynebacterium* oder *Brevibacterium.*

Ein weiterer Aspekt der Erfindung betrifft Verfahren zur Modulation der Produktion eines Moleküls von einem Mikroorganismus. Diese Verfahren umfassen das Zusammenbringen der Zelle mit einer Substanz, die die SES-Proteinaktivität oder die SES-Nukleinsäure-Expression moduliert, so daß eine zellassoziierte Aktivität verglichen mit der gleichen Aktivität bei Fehlen der Substanz verändert wird. Die Zelle wird bei einer bevorzugten Ausführungsform hinsichtlich einer oder mehrerer *C. glutamicum-*Prozesse moduliert, die an der genetischen Stabilität, Genexpression, Proteinfaltung oder Proteinsekretion beteiligt sind, so daß die Ausbeute, Produktion oder Effizienz der Produktion einer gewünschten Feinchemikalien durch diesen Mikroorganismus verbessert wird. Die Substanz, die die SES-Proteinaktivität moduliert, kann eine Substanz sein, die die SES-Proteinaktivität oder die SES-Nukleinsäure-Expression stimuliert. Beispiele für Substanzen, die die SES-Proteinaktivität oder SES-Nukleinsäureexpression stimulieren, umfassen kleine Moleküle, aktive SES-Proteine und Nukleinsäuren, die SES-Proteine codieren und in die Zelle eingebracht worden sind. Beispiele für Substanzen, die die SES-Aktivität oder -Expression hemmen, umfassen kleine Moleküle und SES-Antisense-Nukleinsäuremoleküle.

Ein weiterer Aspekt der Erfindung betrifft Verfahren zur Modulation der Ausbeuten einer gewünschten Verbindung von einer Zelle, umfassend das Einbringen eines SES-Wildtyp- oder -Mutantengens in eine Zelle, das entweder auf einem gesonderten Plasmid bleibt oder in das Genom der Wirtszelle integriert wird. Die Integration in das Genom kann zufallsgemäß oder durch homologe Rekombination erfolgen, so daß das native Gen durch die eingebrachte Kopie ersetzt wird, was die Produktion der gewünschten Verbindung von der zu modulierenden Zelle hervorruft. Diese Ausbeuten sind bei einer bevorzugten Ausführungsform erhöht. Bei einer weiteren bevorzugten Ausführungsform ist die Chemikalie eine Feinchemikalie, die in einer besonders bevorzugten Ausführungsform eine Aminosäure ist. Diese Aminosäure ist in einer besonders bevorzugten Ausführungsform L-Lysin.

### Eingehende Beschreibung der Erfindung

Die vorliegende Erfindung stellt SES-Nukleinsäure- und - Proteinmoleküle bereit, die an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* beteiligt sind. Die erfindungsgemäßen Moleküle könne zur Modulation der Produktion von Feinchemikalien von Mikroorganismen, wie C. *glutamicum*, entweder direkt (z.B. wenn die Überexpression oder Optimierung der Aktivität eines an der Sekretion einer Feinchemikalie beteiligten Proteins (z.B. eines Enzyms) eine direkte Auswirkung auf die Ausbeute, Produktion und/oder Effizienz der Produktion einer Feinchemikalie von den modifizierten C. *glutamicum* hat) oder durch indirekte Auswirkung verwendet werden, die dennoch zu einer Erhöhung der Ausbeute, Produktion und/oder Effizienz der gewünschten Verbindung führt, (z.B. wenn die Modulation der Aktivität oder Kopienzahl eines *C. glutamicum*-DNA-Reparaturproteins zu Änderungen in der Fähigkeit des Mikroorganismus, die eingebrachte Mutation aufrechtzuerhalten, was wiederum die Produktion einer oder mehrerer Feinchemikalien von diesem Stamm beeinflussen kann). Die Aspekte der Erfindung sind nachstehend weiter erläutert.

### I. Feinchemikalien

Der Begriff "Feinchemikalie" ist im Fachgebiet bekannt und beinhaltet Moleküle, die von einem Organismus produziert werden und in verschiedenen Industriezweigen Anwendungen finden, wie bspw., jedoch nicht beschränkt auf die pharmazeutische Industrie, die Landwirtschafts- und Kosmetikindustrie. Diese Verbindungen umfassen organische Säuren, wie Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlehydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malaysia, AOCS Press (1995)), Enzyme und sämtliche anderen von Gutcho (1983) in Chemicals by Fermentation, Noyes Data Corporation, ISBN: 0818805086 und den darin angegebenen Literaturstellen beschriebenen Chemikalien. Der Metabolismus und die Verwendungen bestimmter Feinchemikalien sind nachstehend weiter erläutert.

### A. Metabolismus und Verwendungen von Aminosäuren

Die Aminosäuren umfassen die grundlegenden Struktureinheiten sämtlicher Proteine und sind somit für die normalen Zellfunktionen in allen Organismen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ulmann's Encyclopedia of Industrial Chemistry, Bd. A2, S. 57-97 VCH: Weinheim (1985)). Die Aminosäuren können in der optischen D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L., Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, weil sie aufgrund der Komplexität ihrer Biosynthese gewöhnlich mit der Ernährung aufgenommen werden müssen, werden durch einfache Biosynthesewege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen in Ulmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation s. Umbarger, H.E. (1978) Ann. Rev. Biochem. 47:533-606). Glutamat wird durch reduktive Aminierung von α-Ketoglutarat, einem Zwischenprodukt im CitronensäureZyklus, synthetisiert. Glutamin, Prolin und Arginin werden jeweils nacheinander aus Glutamat erzeugt. Die Biosynthese von Serin erfolgt in einem Dreischritt-Verfahren, beginnt mit 3-Phosphoglycerat (einem Zwischenprodukt der Glykolyse) und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten-β-Kohlenstoffatoms auf Tetrahydrofolat in einer durch Serin-Transhydroxymethylase katalysierten Reaktion. Phenylalanin und Tyrosin werden aus den Vorstufen des Glykolyse- und Pentosephosphatweges, Erythrose-4-phosphat und Phosphoenolpyruvat, in einem 9-Schritt-Biosyntheseweg synthetisiert, der sich nur in den letzten beiden Schritten nach der Synthese von Präphenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf übersteigt, können nicht gespeichert werden, und werden statt dessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über Rückkopplungs-Mechanismen bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### B. Metabolismus und Verwendungen von Vitaminen, Cofaktoren und Nutrazeutika

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenüberträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullman's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nichtproteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullman's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B₆" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)-β-alanin) kann entweder durch chemische Synthese oder durch Fermentation hergestellt werden. Die letzten Schritte bei der Pantothenat-Biosynthese bestehen aus der ATP-getriebenen Kondensation von β-Alanin und Pantoinsäure. Die für die Biosyntheseschritte für die Umwandlung in Pantoinsäure, in β-Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des α-Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien, wie Riboflavin, Vitamin B₆, Pantothenat und Biotin, auch durch großangelegte Anzucht von Mikroorganismen produziert worden sind. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### C. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteile der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisierung zur Bildung von Nukleinsäuremolekülen ist es möglich, die RNA- und DNA-Synthese zu hemmen; wird diese Aktivität zielgerichtet bei Krebszellen gehemmt, läßt sich die Teilungs- und Replikationsfähigkeit von Tumorzellen hemmen. Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10:505-548). Untersuchungen an Enzymen, die am Purin- und Pyrimidin-Metabolismus beteiligt sind, haben sich auf die Entwicklung neuer Medikamente konzentriert, die bspw. als Immunsuppressiva oder Antiproliferantien verwendet werden können (Smith, J.L. (1995) "Enzymes in Nucleotide Synthesis" Curr. Opin. Struct. Biol. 5:752-757; (1995) Biochem. Soc. Transact. 23:877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, die gewöhnlich als Geschmacksverstärker (bspw. IMP oder GMP) oder für viele medizinische Anwendungen verwendet werden (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden, entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular Biology, Bd. 42, Academic Press, S. 259-287; und Michal, G. (1999) "Nucleotides and Nucleosides"; Kap. 8 in: Biochemical Pathways: An Atlas of Biochemistry and Molecular Biology, Wiley, New York). Der Purin-Metabolismus, das Objekt intensiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden aus Ribose-5-phosphat über eine Reihe von Schritten über die Zwischenverbindung Inosin-5'-phosphat (IMP) synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### D. Trehalose-Metabolismus und Verwendungen

Trehalose besteht aus zwei Glucosemolekülen, die über eine α,α-1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. (1998) Trends Biotech. 16:460-467; Paiva, C.L.A. und Panek, A.D. (1996) Biotech Ann. Rev. 2:293-314; und Shiosaka, M. (1997) J. Japan 172:97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

### II. Genetische Stabilität, Proteinsynthese und Proteinsekretion in C. glutamicum

Die Produktion einer gewünschten Verbindung von einer Zelle, wie *C. glutamicum*, ist die Kulmination einer großen Zahl an getrennten und trotzdem miteinander verknüpften Prozessen, von denen jeder für die Gesamtproduktion und die Freisetzung der Verbindung aus der Zelle entscheidend ist. Bei der Veränderung einer Zelle, so daß sie eine oder mehrere Chemikalien überproduziert, muß jeder dieser Prozesse berücksichtigt werden, um zu gewährleisten, daß die biochemische Maschinerie der Zelle mit dieser genetischen Manipulation kompatibel ist. Besonders bedeutende zelluläre Mechanismen umfassen die Stabilität das/der veränderten Gen(s/e) beim Einbringen in die Zelle, die Fähigkeit der mutierten Gens, richtig transkribiert und translatiert zu werden (einschließlich der Codonverwendung) und die Fähigkeit des mutierten Proteinproduktes, richtig gefaltet und/oder sezerniert zu werden.

### A. Bakterielle Reparatur- und Rekombinationssysteme

Zellen sind ständig nukleinsäureschädigenden Agenzien, wie UV-Bestrahlung, Sauerstoffradikale und Alkylierung, ausgesetzt. Ferner ist sogar die Wirkung von DNA-Polymerasen nicht fehlerfrei. Die Zellen müssen ein Gleichgewicht zwischen der genetischen Stabilität (die gewährleistet, daß Gene, die für zelluläre Funktionen notwendig sind, nicht während des normalen Wachstums und Stoffwechsels beschädigt werden) und der genetische Variabilität(die es den Zellen ermöglicht, sich an eine sich ändernde Umwelt anzupassen) aufrechterhalten. Daher gibt es in den meisten Zellen getrennte, aber miteinander zusammenhängende Wege für die DNA-Reparatur und DNA-Rekombination. Ersterer dient der strikten Korrektur von Fehlern in DNA-Molekülen durch entweder das direkte Rückgängigmachen der Schädigung oder durch Ausschneiden des geschädigten Bereichs und Ersetzen durch die korrekte Sequenz. Das letztere Rekombinationssystem repariert auch Nukleinsäuremoleküle, aber nur solche Schäden, die zu einer Schädigung in beiden DNA-Strängen führen, so daß kein Strang als Matrize zur Korrektur des anderen verwendet werden kann. Die Rekombinationsreparatur und die SOS-Reaktion können leicht zu Inversionen, Deletionen oder anderen genetischen Umlagerungen innerhalb des oder um den beschädigten Bereich führen, was wiederum einen bestimmten Grad an genomischer Instabilität fördert, der zur Fähigkeit der Zelle, sich an ändernde Umgebungen oder Streß anzupassen, beitragen kann.

High-fidelity-Reparaturmechanismen beinhalten das direkte Rückgängigmachen des DNA-Schadens und das Ausschneiden des Schadens und die Resynthese unter Verwendung der im Gegenstrang codierten Information. Das direkte Rückgängigmachen des Schadens erfordert ein Enzym mit einer Aktivität, die das Gegenteil desjenigen bewirkt, was ursprünglich die DNA beschädigt hat. Beispielsweise kann eine unrichtige Methylierung von DNA durch die Wirkung von DNA-Reparatur-Methyltransferasen korrigiert werden, und durch UV-Bestrahlung erzeugte Nukleotiddimere können durch die Aktivität der Desoxyribodipyrimidinphotolyase repariert werden, die in Gegenwart von Licht das Dimer wieder in die entsprechenden Nukleotide spaltet (s. Michal, G. (1999) Biochemical Pathways: An Atlas of Biochemistry and Molecular Biology, Wiley: New York, und die darin zitierten Literaturstellen).

Die genaue Reparatur größerer Schäden erfordert spezialisierte Reparaturmechanismen. Dazu gehören die Mismatch-Reparatur- und die Ausschneide-Reparatursysteme. Die Beschädigung einer einzelnen Base kann durch eine Reihe von Spaltungsreaktionen korrigiert werden, wobei zuerst die Zuckerbindung gespalten wird, gefolgt von Spaltung des DNA-Rückgrats an der beschädigten Stelle und Entfernen der beschädigten Base selbst. Schließlich bewirken DNA-Polymerase und DNA-Ligase das Auffüllen und Versiegeln der Lücke unter Verwendung des zweiten DNA-Strangs als Matrize. Ein erheblicherer DNA-Schaden, der zu einer veränderten Konformation der Doppelhelix führt, wird durch das ABC-System korrigiert, bei dem Helicase II, DNA-Polymerase I, die UvrA-, UvrB- und UvrC-Proteine zusammen die Doppelhelix an der beschädigten Stelle einzelsträngig spalten, den beschädigten Bereich auf ATP-abhängige Weise aufwinden, den beschädigten Bereich ausschneiden und den fehlenden Bereich mit dem anderen Strang als Matrize auffüllen. Schließlich versiegelt die DNA-Ligase den Einzelstrangbruch. Spezifische Reparatursysteme gibt es auch für G-T-Mismatches (an denen das Vsr-Protein beteiligt ist) und für kleine Deletions-/Insertionsfehler aufgrund der falschen Reparatur der beiden Stränge (an denen der methylierungsgesteuerte Weg beteiligt ist).

Es gibt auch Low-fidelity-Reparatursysteme, die gewöhnlich zur Korrektur sehr ausgedehnter DNA-Schäden bei Bakterien verwendet werden. Doppelstrangreparatur und Rekombination erfolgen bei Vorliegen einer Schädigung, die beide DNA-Stränge betrifft. In dieser Situation ist es unmöglich, den Schaden unter Verwendung des anderen Strangs als Matrize zu reparieren. Somit beinhaltet das Reparatursystem ein Doppel-Crossover-Ereignis zwischen dem beschädigten Bereich und einer anderen Kopie des Bereichs auf einem homologen DNA-Molekül. Dies ist möglich, da sich Bakterien so schnell teilen, daß eine zweite Kopie der genomischen DNA gewöhnlich verfügbar ist, bevor die Zellteilung tatsächlich stattfindet. Dieses Crossover-Ereignis kann leicht zu Inversionen, Duplikationen, Deletionen, Insertionen und anderen genetischen Umlagerungen führen und erhöht so insgesamt die genetische Instabilität des Organismus.

Die SOS-Reaktion wird aktiviert, wenn eine ausreichende Schädigung in der DNA vorliegt, daß die DNA-Polymerase III anhält und nicht mit der Replikation fortfahren kann. Unter diesen Umständen ist einzelsträngige DNA zugegen. Das RecA-Protein wird durch Bindung an einzelsträngige DNA aktiviert, und diese aktivierte Form führt zur Aktivierung des LexA-Repressors, wodurch der Transkriptionsblock von mehr als 20 Genen aufgehoben wird, einschließlich UvrA, UvrB, UvrC, Helicase II, DNA pol III, UmuC und UmuD. Die kombinierten Aktivitäten dieser Enzyme bewirken ein ausreichendes Auffüllen des Lückenbereichs, daß DNA pol III die Replikation wieder aufnehmen kann. Diese Lücken werden jedoch mit Basen aufgefüllt, die nicht vorliegen sollten; somit führt dieser Reparaturtyp zu einer fehleranfälligen Reparatur, was insgesamt zur genetischen Instabilität in der Zelle beiträgt.

### B. Transposons

Die oben genannten Systeme mit High- oder Low-fidelity sollen DNA-Schäden reparieren. Unter bestimmten Umständen kann diese Reparatur zusätzliche Genumlagerungen umfassen. Viele Bakterienzellen haben außerdem Mechanismen, die spezifisch solche Genumlagerungen verursachen sollen. Besonders gut bekannte Beispiele für solche Mechanismen sind die Transposons.

Transposons sind genetische Elemente, die von einer Stelle zu einer anderen entweder innerhalb eines Chromosoms oder zwischen einem Stück extrachromosomaler DNA (z.B. einem Plasmid) und einem Chromosom wandern können. Die Transposition auf auf mehrere Weisen erfolgen; beispielsweise kann das transponierbare Element aus der Donorstelle ausgeschnitten und in die Zielstelle integriert werden (nicht-replikative Transposition), oder das transponierbare Element kann alternativ von der Donorstelle zur Zielstelle dupliziert werden, was zwei Kopien des Elements ergibt (replikative Transposition). Gewöhnlich gibt es keine Sequenzverwandtschaft zwischen der Donor- und der Zielstelle.

Dieses Transpositionsereignis hat eine Vielzahl möglicher Ergebnisse. Die Integration eines transposablen Elementes in ein Gen disrumpiert das Gen, was dessen Funktion gewöhnlich völlig ausschaltet. Ein Integrationsereignis, das in der das Gen umgebenden DNA stattfindet, kann nicht die codierende Sequenz selbst stören, aber eine grundlegende Auswirkung auf die Regulation des Gens und somit auf seine Expression haben. Rekombinationsereignisse zwischen zwei Kopien eines transposablen Elementes, das sich in verschiedenen Abschnitten des Genoms befindet, können zu Deletionen, Duplikationen, Inversionen, Transpositionen oder Amplifikationen von Segmenten des genoms führen. Es ist auch möglich, daß verschiedene Replikons fusionieren.

Die einfachsten transposonartigen genetischen Elemente werden als Insertions- (IS-) Elemente bezeichnet. IS-Elemente enthalten einen Nukleotidbereich variabler Länge (aber gewöhnlich weniger als 1500 Basen), der keine codierenden Bereiche enthält und an jedem ende von Inverted Repeats umgeben ist. Da das IS-Element keine Proteine codiert, deren Aktivität nachgewiesen werden kann, wird das Vorliegen eines IS-Elementes gewöhnlich nur aufgrund eines Funktionsverlustes von einem oder mehreren Genen, in die das IS-Element inseriert ist, beobachtet.

Transposons sind mobile genetische Elemente, die im Gegensatz zu IS-Elementen von Repeats begrenzte Nukleinsäuresequenzen enthalten, die ein oder mehrere Proteine codieren können. Es ist nicht ungewöhnlich, daß diese Repeatbereiche aus IS-Elementen bestehen. Die vom Transposon codierten Proteine sind gewöhnlich Transposasen (Proteine, die die Wanderung des Transposons von einer Stelle zur anderen katalysieren) und Antibiotika-Resistenzgene. Die Mechanismen und die Regulation der transposablen Elemente sind im Fachgebiet bekannt und wurden zumindest bspw. beschrieben in: Lengeler et al. (1999) Biology of Prokaryotes, Thieme Verlag: Stuttgart, S. 375-361; Neidhardt et al. (1996) *Escherichia coli* and *Salmonella,* ASM Press: Washington, D.C.; Sonenshein, Al.L., et al., Hrsg. (1993) *Bacillus subtilis,* ASM Press, Washington, D.C.; Voet, D., und Voet, J.G. (1992) Biochemie, VCH: Weinheim, S. 985-990; Brock, T.D., und Madigan, M.T. (1991) Biology of Mocroorganisms, 6. Aufl. Prentice Hall: New York, S. 267-269; und Kleckner, N. (1990) "Regulation of transposition in bacteria", Annu. Rev. Biochem. 61:297-327.

### C. Transkription

Die Genexpression in Bakterien wird hauptsächlich auf der Ebene der Transkription reguliert. Der Transkriptionsapparat besteht aus einer Reihe von Proteinen, die man in zwei Gruppen einteilen kann: RNA-Polymerase (das operierende DNA-transkribierende Enzym) und Sigma-Faktoren (die die Gentranskription regulieren, indem sie die RNA-Polymerase zu spezifischer Promotor-DNA-Sequenzen lenken, die diese Faktoren erkennen). Die Kombination von RNA-Polymerase und Sigma-Faktoren erzeugt das RNA-Polymerase-Holoenzym, einen aktivierten Komplex. Gram-positive Bakterien, wie Corynebakterien, enthalten nur einen Typ der RNA-Polymerase, aber eine Anzahl verschiedener Sigma-Faktoren, die für verschiedene Promotoren, Wachstumsphasen, Umweltbedingungen, Substrate, Sauerstoffspiegel, Transportprozesse und dgl. Spezifisch sind, wodurch sich der Organismus an verschiedene Umwelt-und Stoffwechselbedingungen anpassen kann.

Promotoren sind spezifische DNA-Sequenzen, die als Andockstellen für das RNA-Polymerase-Holoenzym dienen. Viele Promotorelemente besitzen konservierte Sequenzelemente, die durch Homologiesuchen erkannt werden können; alternativ können Promotorbereiche für ein bestimmtes Gen unter Verwendung von Standard-Techniken, wie Primerextension, identifiziert werden. Viele Promotorbereiche von gram-positiven Bakterien sind bekannt (s. z.B. Sonenshein, A.L., Hoch, J.A., und Losick, R., Hrsg. (1993) *Bacillus subtilis,* ASM Press: Washington, D.C.).

Die Promotor-Transkriptionskontrolle wird durch mehrere Repressions- oder Aktivierungsmechanismen beeinflußt. Spezifische regulatorische Proteine, die an Promotoren binden, haben die Fähigkeit, die Bindung des RNA-Holoenzyms zu blockieren (Repressoren) oder diese zu unterstützen (Aktivatoren) und so die Transkription zu regulieren. Die Bindung dieser Repressor- und Aktivatormoleküle wird wiederum durch ihre Wechselwirkungen mit anderen Molekülen, wie Proteinen oder anderen Stoffwechselverbindungen, reguliert. Die Transkription kann alternativ durch Faktoren reguliert werden, die Prozesse, wie die Elongation oder Termination beeinflussen (s. z.B. Sonenshein, A.L., Hoch, J.A., und Losick, R., Hrsg. (1993) *Bacillus subtilis,* ASM Press: Washington, D.C.). Durch die Fähigkeit, die Transkription von Genen als Reaktion auf eine Vielzahl von Umwelt- oder Stoffwechselzeichen zu regulieren, können die Zellen genau steuern, wann ein Gen exprimiert werden kann und wieviel eines Genproduktes in der Zelle zu einem Zeitpunkt vorliegen kann. Dies verhindert wiederum die unnötige Verschwendung von Energie oder die unnötige Verwendung möglicherweise rarer Zwischenverbindungen oder Cofaktoren.

### D. Translation und Aminoacyl-tRNA-Synthetasen

Die Translation ist der Prozeß, durch den ein Polypeptid aus Aminosäuren gemäß der in einem RNA-Molekül enthaltenen Information synthetisiert wird. Die Hauptkomponenten dieses Prozesses sind Ribosomen und spezifische Initiations- oder Elongationsfaktoren, wie IF1-3, ERFINDUNGSGEMÄSS-G und EFTu (s. z.B. Sonenshein, A.L., Hoch, J.A., und Losick, R., Hrsg. (1993) *Bacillus subtilis,* ASM Press: Washington, D.C.).

Jedes Codon des mRNA-Moleküls codiert eine bestimmte Aminosäure. Die Umwandlung von mRNA in Aminosäure wird durch Transfer-RNA-(tRNA-) Moleküle durchgeführt. Diese Moleküle bestehen aus einem RNA-Einzelstrang (zwischen 60 und 100 Basen), der in einer L-förmigen dreidimensionalen Struktur mit hinausragenden Bereichen oder "Armen" vorliegt. Einer dieser Arme bildet Basenpaare mit einer bestimmten Codonsequenz auf dem mRNA-Molekül. Ein zweiter Arm interagiert spezifisch mit einer bestimmten Aminosäure (die vom Codon codiert wird). Andere tRNA-Arme umfassen den variablen Arm, den TΨC-Arm (der Thymidylat- und Pseudouridylatmodifikationen trägt) und den D-Arm (der eine Dihydrouridinmodifikation trägt). Die Funktion dieser letzteren Strukturen ist immer noch unbekannt, aber ihre Konservierung zwischen den tRNA-Molekülen legt eine Rolle bei der Proteinsynthese nahe.

Damit das auf Nukleinsäure basierende tRNA-Molekül sich mit der korrekten Aminosäure paart, muß eine Familie von Enzymen, die als Aminoacyl-tRNA-Synthetasen bezeichnet werden, wirken. Es gibt viele verschiedene dieser Enzyme, und jedes ist spezifisch für eine bestimmte tRNA und eine bestimmte Aminosäure. Diese Enzyme binden das 3'-Hydroxyl der endständigen tRNA-Adenosin-Ribose-Einheit in einer Zwei-Schritt-Reaktion an die Aminosäure. Zuerst wird das Enzym durch Reaktion mit ATP und der Aminosäure aktiviert, woraus ein Aminoacyl-tRNA-Synthetase-Aminoacyl-Adenylat-Komplex resultiert. Zweitens wird die Aminoacylgruppe vom Enzym auf die Ziel-tRNA übertragen, an der sie in einem energiereichen Zustand bleibt. Die Bindung des tRNA-Moleküls an sein Erkennungscodon auf dem mRNA-Molekül bringt dann die an die tRNA gebundene energiereiche Aminosäure in Kontakt mit dem Ribosom. Innerhalb des Ribosoms besetzt die Aminosäure-beladene tRNA (Aminoacyl-tRNA) eine Bindungsstelle (die A-Stelle) neben einer zweiten Stelle (der P-Stelle), die ein tRNA-Molekül trägt, dessen Aminosäure an die naszierende Polypeptidkette gebunden ist (Peptidyl-tRNA). Die aktivierte Aminosäure an der Aminoacyl-tRNA ist ausreichend reaktiv, daß sich spontan eine Peptidbindung zwischen dieser Aminosäure und der nächsten Aminosäure an der naszierenden Polypeptidkette bildet. Die GTP-Hydrolyse liefert die Energie zum Transfer der jetzt mit der Polypeptidkette beladenen tRNA von der A-Stelle zur P-Stelle des Ribosoms, und der Prozeß wiederholt sich, bis ein Stopcodon erreicht wird.

Es gibt eine Reihe verschiedener Schritte, an denen die Translation reguliert werden kann. Dazu gehören die Bindung des Ribosoms an mRNA, das Vorliegen von mRNA-Sekundärstruktur, die Codonverwendung oder die Häufigkeit bestimmter tRNAs. Auch spezielle Regulationsmechanismen, wie Attenuation, können auf der Translationsebene wirken. Eine tiefgreifende Übersicht über viele dieser Mechanismen s. z.B. in Vellanoweth, R.L. (1993) "Translation and its Regulation", in: *Bacillus subtilis* and o-ther Gram Positive Bacteria, Sonenshein, A.L., et al., Hrsg., ASM Press: Washington, D.C., S. 699-711 und die darin zitierten Literaturstellen.

### E. Proteinfaltung und -Sekretion

Die Synthese von Proteinen durch das Ribosom führt zu Polypeptidketten, die eine dreidimensionale Form annehmen müssen, bevor das Protein normal funktionieren kann. Die dreidimensionale Struktur wird durch einen Faltungsprozeß erzielt. Polypeptidketten sind flexibel und bewegen sich (im Prinzip) leicht und frei in Lösung, bis sie eine Konformation annehmen, die zu einer stabilen dreidimensionalen Struktur führt. Manchmal ist es jedoch für Proteine schwierig, sich richtig zu falten, entweder aufgrund der Umweltbedingungen (z.B. hohe Temperatur, bei der die im System vorhandene kinetische Energie es dem Protein schwieriger macht, in das Energieloch einer stabilen Struktur zu fallen) oder aufgrund der Art des Proteins selbst (z.B. neigen hydrophobe Bereiche in nahe beieinander befindlichen Proteinen zur Aggregation, wodurch sie sich selbst aus wäßrigen Lösungen ausfällen).

Proteinartige Faktoren sind identifiziert worden, die die Faltung von Proteinen katalysieren, begleiten oder anderweitig unterstützen können und co- oder posttranslational synthetisiert werden. Zu diesen Proteinfaltungsmolekülen gehören die Prolyl-Peptidyl-Isomerasen (z.B. Trigger-Faktor, Cyclophilin und FKBP-Homologa) sowie Proteine der Hitzeschockprotein-Gruppe (z.B. DnaK, DnaJ, GroEL, kleine Hitzeschockproteine, HtpG und Mitglieder der Clp-Familie (z.B. ClpA, ClpB, ClpW, ClpP und ClpX). Viele dieser Proteine sind für die Lebensfähigkeit von Zellen wichtig: zusätzlich zu ihrer Funktion bei der Proteinfaltung, - translokation und -prozessierung dienen sie häufig als Ziele für die Gesamtregulation der Proteinsynthese (s. z.B. Bukau, B. (1993) Molecular Microbiology 9(4):671-680; Bukau, B., und Horwich, A.L. (1998) Cell 92(3):351-366; Hesterkamp, T., Bukau, C. (1996) FEBS Lett. 389(1):32-34; Yaron, A., Naider, F. (1993) Critical Reviews in Biochemistry and Molecular Biology 28(1):31-81; Scheibel, R., Buchner, J. (1998) Biochemical Pharmacology 56(6):675-682; Ellis, R.J., Hartl, F.U. (1996) FASEB Journal 10(1):20-26; Wawrzynow, A., et al. (1996) Molecular Micorbiology 21(5):895-899; Ewalt, K.L., et al. (1997) Cell 90(3):491-500).

Die bisher identifizierten Chaperone wirken auf zwei Weisen: sie binden entweder an Polypeptide und stabilisieren diese oder sie stellen eine Umgebung bereit, in der die Faltung ohne Störung stattfinden kann. Die erstere Gruppe, einschließlich z.B. DnaK, DnaJ und der Hitzeschockproteine, bindet direkt an das naszierende oder falsch gefaltete Polypeptid, häufig begleitet von ATP-Hydrolyse. Die Bindung des Chaperons verhindert, daß das Polypeptid mit anderen Polypeptiden aggregiert, und kann die Auflösung dieser Aggregate, wenn sie sich bereits gebildet haben, erzwingen. Nach der Wechselwirkung mit einem zweiten Chaperon GrpE (das das Auftreten eines ADP-ATP-Austauschs ermöglicht) wird das Polypeptid im Molten-globule-Zustand freigesetzt und kann sich falten. Wenn eine falsche Faltung auftritt, binden die Chaperone wieder an das falsch gefaltete Protein und erzwingen seine Rückkehr in einen ungefalteten Zustand. Dieser Zyklus kann wiederholt werden, bis das Protein korrekt gefaltet ist. Im Gegensatz zur ersten Chaperongruppe, die einfach an das Polypeptid bindet, bindet die zweite Gruppe (z.B. GroEL/ES) nicht nur an das Polypeptid, sondern umgibt es vollständig, so daß es vor der Umgebung geschützt ist. Der GroEL/ES-Komplex besteht aus zwei aufeinandergestapelten 14-gliedrigen Ringen mit einer hydrophoben inneren Oberfläche und einem "Deckel" aus einem 7-gliedrigen Ring. Das Polypeptid wird in einer ATP-abhängigen Reaktion in den Kanal im Zentrum dieses Komplexes gezogen, wo es sich ohne Störung durch andere Polypeptide falten kann. Falsch gefaltete Proteine werden nicht aus dem Komplex freigesetzt.

Ein wichtiger Schritt bei der Proteinfaltung ist die Bildung von Disulfidbindungen. Diese Bindungen, entweder innerhalb einer Untereinheit oder zwischen Untereinheiten von Proteinen, sind für die Proteinstabilität wichtig. Disulfidbindungen bilden sich leicht in wäßriger Lösung, und es ist schwierig, eine falsche Disulfidbrückenbildung ohne Hilfe einer reduzierenden Umgebung rückgängig zu machen. Zur Unterstützung dieses Prozesses der korrekten Disulfidbrückenbildung findet man im Cytosol der meisten Zellen thiolhaltige Moleküle, wie Glutathion oder Thioredoxin und ihre entsprechenden Oxidations-/Reduktionssysteme (Loferer, H., Hennecke, H. (1994) Trends in Biochemical Sciences 19 (4) :169-171) .

Zu bestimmten Zeiten ist jedoch die Faltung naszierender Polypeptidkette nicht wünschenswert, bspw. wenn diese Proteine sezerniert werden sollen. Der Faltungsprozeß führt gewöhnlich dazu, daß die hydrophoben Bereiche des Proteins sich im Zentrum des Proteins, entfernt von der wäßrigen Lösung, befinden und die hydrophilen Bereiche an den äußeren Oberflächen des Proteins präsentiert werden. Diese Konformationsanordnung erzeugt zwar eine höherer Stabilität für das Protein, erschwert aber die Translokation des Proteins über Membranen, da der hydrophobe Kern der Membran an sich inkompatibel mit dem hydrophilen Äußeren des Proteins ist. So werden die von der Zelle synthetisierten Proteine, die zum Äußeren der Zelle sezerniert werden müssen (z.B. Zelloberflächenenzyme und Membranrezeptoren) oder die in die Membran selbst inseriert werden müssen (z.B. Transporterproteine und Kanalproteine), gewöhnlich vor der Faltung sezerniert oder inseriert. Die gleichen Chaperone, die die Aggregation naszierender Polypeptidketten verhindern, verhindern auch die Faltung von Polypeptiden, bis sie nicht mehr gebraucht werden. Somit können diese Proteine naszierende Polypeptidketten zu einem geeigneten Ort in der Zelle "eskortieren", wo sie entweder entfernt werden, so daß die Faltung möglich wird, oder das Protein auf ein Transportsystem übertragen, das entweder das Polypeptid sezerniert oder seine Insertion in eine Membran unterstützt.

Im Verlauf der Evolution hat sich eine spezialisierte Proteinmaschinerie gebildet, die Proteine mit spezifischen Prosequenzen (die später durch Spaltung aus dem Protein entfernt werden) erkennt, bindet, Transportiert und prozessiert. Die Maschinerie besteht aus einer Reihe von Proteinen, die man gemeinsam als sec (Typ-II-Sekretions-) System bezeichnet (eine Übersicht s. in Gilbert, M., et al. (1995) Critical Reviews in Biotechnology 15(1):13-39 und den Literaturstellen darin; Freudl, R. (1992) Journal of Biotechnology 23(3):231-240 und Literaturstellen darin; Neidhardt, F.C., et al. (1996) *E. coli* and *Salmonella,* ASM Press: Washington, D.C., S. 967-978; Binet, R., et al. (1997) Gene 192(1):7-11 und Rapoport, T.A. (1986) Critical Reviews in Biochemistry 20(1):73-137 und Literaturstellen darin). Das sec-System besteht aus Chaperonen (z.B. SecA und SecB), integralen Membranproteinen, die auch als Translokasen bezeichnet werden (z.B. SecY, SecE und SecG) und Signalpeptidasen (z.B. LepB). Das naszierende Polypeptid mit einer prosequenz, die zur Sekretion führt, wird von SecB gebunden, das es an SecA an der inneren Oberfläche der Zellmembran übergibt. SecA bindet an die Prosequenz und inseriert nach ATP-Hydrolyse in die Membran und zwingt auch einen Teil des Polypeptids durch die Membran. Der Rest des Polypeptids wird durch einen Komplex aus Translokasen, wie SecY, SecE und SecG, durch die Membran geleitet. Schließlich spaltet die Signalpeptidase die Prosequenz ab, und das Polypeptid befindet sind frei auf der extrazellulären Seite der Membran, wo es sich spontan faltet.

Auch Sec-unabhängige Sekretionsmechanismen sind bekannt. Beispielsweise umfaßt der Signalerkennungspartikel-abhängige Weg die Bindung eines Signalerkennungspartikel- (SRP-) Proteins an das naszierende Polypeptid während seiner Synthese, wodurch das Ribosom anhält. Ein Rezeptor für SRP an der inneren Oberfläche der Membran bindet dann den Ribosom-Polypeptid-SRP-Komplex. GTP-Hydrolyse liefert die Energie, die zur Übertragung des Komplexes auf den sec-Translokase-Komplex nötig ist, an dem das Polypeptid während seiner Synthese durch das Ribosom über die Membran geleitet wird. Bekanntlich existieren andere, für nur wenige Proteine spezifische Sekretionsmechanismen.

### III.Elemente und Verfahren der Erfindung

Die vorliegende Erfindung beruht zumindest teilweise auf der Entdeckung von neuen Molekülen, die hier als SES-Nukleinsäure-und Proteinmoleküle bezeichnet werden und an der Reparatur oder Rekombination von DNA in C. *glutamicum,* Transposition oder anderen Umlagerung von *C. glutamicum*-DNA, Genexpression in *C. glutamicum* (d. h. Transkriptions- oder Translationsprozessen), Proteinfaltung oder Proteinsekretion diese Mikroorganismus teilnehmen. In einer Ausführungsform nehmen die SES-Moleküle an der Reparatur oder Rekombination von DNA, Transposition von genetischem Material, Genexpression (d. h. Transkriptions- oder Translationsprozessen), Proteinfaltung oder Proteinsekretion in Cory*nebacterium glutamicum* teil. In einer bevorzugten Ausführungsform hat die Aktivität der erfindungsgemäßen SES-Moleküle bezüglich der Reparatur oder Rekombination von DNA, Transposition von DNA, Genexpression, Proteinfaltung oder Proteinsekretion eine Auswirkung auf die Produktion einer gewünschten Feinchemikalie durch diesen Mikroorganismus. In einer besonders bevorzugten Ausführungsform ist die Aktivität der erfindungsgemäßen SES-Moleküle moduliert, so daß auch die Aktivität der *C. glutamicum-*Zellprozesse, an denen die erfindungsgemäßen SES-Proteine beteiligt sind, (z.B. Reparatur oder Rekombination von DNA, Transposition von DNA, Genexpression, Proteinfaltung oder Proteinsekretion) verändert ist, was direkt oder indirekt zu einer Modulation der Ausbeute, Produktion und/oder Effizienz der Produktion einer gewünschten Feinchemikalie durch C. *glutamicum* führt.

Der Begriff "SES-Protein" oder "SES-Polypeptid" umfaßt Proteine, die an einer Reihe von Zellprozessen beteiligt sind, die zur genetischen Stabilität, Genexpression, Proteinfaltung oder Proteinsekretion von C. *glutamicum* in Beziehung stehen. Beispielsweise kann ein SES-Protein an der DNA-Reparatur oder an Rekombinationsmechanismen bei C. *glutamicum,* Umlagerungen des genetischen Materials von C. *glutamicum* (wie den von Transposons vermittelten), der Transkription oder Translation von Genes in diesem Mikroorganismus, bei der Vermittlung der Proteinfaltung in C. *glutamicum* (wie der Aktivität von Chaperonen) oder der Sekretion von Proteinen aus C. glutamicum-Zellen (z.B. am sec-System) beteiligt sein. Beispiele für SES-Proteine umfassen solche, die von den in Tabelle 1 und Anhang A aufgeführten SES-Genen codiert werden. Die Ausdrücke "SES-Gen" oder "SES-Nukleinsäuresequenz" umfassen Nukleinsäuresequenzen, die ein SES-Protein codieren, das aus einem codierenden Bereich und entsprechenden untranslatierten 5'- und 3'-Sequenzbereichen besteht. Beispiele für SES-Gene sind die in Tabelle 1 aufgelisteten. Die Begriffe "Produktion" oder "Produktivität" sind im Fachgebiet bekannt und beinhalten die Konzentration des Fermentationsproduktes (bspw. der gewünschten Feinchemikalie), das innerhalb einer festgelegten Zeitspanne und eines festgelegten Fermentationsvolumens gebildet wird (bspw. kg Produkt pro Std. pro 1). Der Begriff "Effizienz der Produktion" umfaßt die Zeit, die zur Erzielung einer bestimmten Produktionsmenge nötig ist (bspw. wie lange die Zelle zur Aufrichtung einer bestimmten Ausstoßrate einer Feinchemikalie benötigt). Der Begriff "Ausbeute" oder "Produkt/Kohlenstoff-Ausbeute" ist im Fachgebiet bekannt und umfaßt die Effizienz der Umwandlung der Kohlenstoffquelle in das Produkt (d.h. die Feinchemikalie). Dies wird bspw. gewöhnlich ausgedrückt als kg Produkt pro kg Kohlenstoffquelle. Durch Vergrößern der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe "Biosynthese" oder "Biosyntheseweg" sind im Fachgebiet bekannt und umfassen die Synthese einer Verbindung, vorzugsweise einer organischen Verbindung, durch eine Zelle aus Zwischenverbindungen, bspw. in einem Mehrschritt-oder stark regulierten Prozeß. Die Begriffe "Abbau" oder "Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle), bspw. in einem Mehrschritt- oder stark regulierten Prozeß. Der Begriff "Metabolismus" ist im Fachgebiet bekannt und umfaßt die Gesamtheit der biochemischen Reaktionen, die in einem Organismus stattfinden. Der Metabolismus einer bestimmten Verbindung (z.B. der Metabolismus einer Aminosäure, wie Glycin) umfaßt dann sämtliche Biosynthese-, Modifikations- und Abbauwege in der Zelle, die diese Verbindung betreffen. Der Begriff "DNA-Reparatur" ist im Fachgebiet bekannt und beinhaltet zelluläre Mechanismen, durch die Fehler in der DNA (entweder aufgrund von Schäden, wie, aber nicht beschränkt auf Ultraviolettbestrahlung, Methylasen, Low-fidelity-Replikation oder Mutagene) ausgeschnitten und korrigiert werden. Der Ausdruck "Rekombination" oder "DNA-Rekombination" ist im Fachgebiet bekannt und umfaßt zelluläre Mechanismen, durch die ausgedehnte DNA-Schäden, die beide Stränge eines DNA-Moleküls betreffen, durch homologe Rekombination mit einer anderen unbeschädigten Kopie des DNA-Moleküls innerhalb der gleichen Zelle korrigiert werden. Diese Reparaturen sind gewöhnlich low-fidelity und können zu Genumlagerungen führen. Der Begriff "Transposon" ist im Fachgebiet bekannt und umfaßt ein DNA-Element, das zufallsgemäß in das Genom eines Organismus inserieren kann und zur Disruption von Genen oder ihrer regulatorischen Bereiche oder zu Duplikationen, Inversionen, Deletionen und anderen Genumlagerungen führen kann. Der Begriff "Proteinfaltung" ist im Fachgebiet bekannt und umfaßt die Wanderung einer Polypeptidkette durch mehrere dreidimensionale Konfigurationen, bis die stabile, aktive, dreidimensionale Konfiguration erzielt wird. Die Bildung von Disulfidbindungen und die Sequestrierung hydrophober Bereich aus der umgebenden wäßrigen Lösung liefern einige der Antriebskräfte für diesen Proteinfaltungsprozeß, und die korrekte Faltung kann durch die Aktivität von Chaperonen verstärkt werden. Die Begriffe "Sekretion" oder "Proteinsekretion" sind im Fachgebiet bekannt und umfassen die Bewegung von Proteinen vom Inneren der Zelle zum Äußeren der Zelle in einem Mechanismus, bei dem ein System von Sekretionsproteinen ihren Durchtritt über die Zellmembran zum Äußeren der Zelle ermöglicht.

Die erfindungsgemäßen SES-Moleküle sind in einer anderen Ausführungsform befähigt, die Produktion eines gewünschten Moleküls, wie einer Feinchemikalie, in einem Mikroorganismus, wie C. *glu*tamicum, zu modulieren. Es gibt eine Reihe von Mechanismen, durch die die Veränderung eines erfindungsgemäßen SES-Proteins die Ausbeute, Produktion und/oder Effizienz der Produktion einer Feinchemikalie aus einem C. glutamicum-Stamm, der dieses veränderte Protein enthält, direkt beeinflussen kann. Zum Beispiel sollte die Modulation von Proteinen, die direkt an der Transkription oder Translation beteiligt sind (z.B. Polymerasen oder Ribosomen), so daß ihre Anzahl oder Aktivität gesteigert wird, die zelluläre Transkription oder Translation (oder die Geschwindigkeiten dieser Prozesse) insgesamt steigern. Diese erhöhte zelluläre Genexpression sollte solche Proteine umfassen, die an der Feinchemikalienbiosynthese beteiligt sind, so daß eine Steigerung der Ausbeute, Produktion oder Effizienz der Produktion einer oder mehrerer gewünschten Verbindungen erfolgen kann. Modifikationen der Transkriptions-/Translations-Proteinmaschinerie von *C. glutamicum*, so daß die Regulation dieser Proteine verändert wird, kann auch die erhöhte Expression von Genen, die an der Produktion von Feinchemikalien beteiligt sind, ermöglichen. Die Modulation der Aktivität einer Reihe von Proteinen, die an der Peptidfaltung beteiligt sind, kann eine Erhöhung der Gesamtproduktion korrekt gefalteter Moleküle in der Zelle ermöglichen, wodurch die Möglichkeit erhöht wird, daß gewünschte Proteine (z.B. Feinchemikalienbiosynthese-Proteine) richtig funktionieren können. Ferner kann es durch Mutation von an der Sekretion aus *C. glutamicum* beteiligten Proteinen, so daß ihre Anzahl oder Aktivität erhöht ist, möglich sein, die Sekretion einer Feinchemikalie (z.B. eines Enzyms) aus Zellen in der Fermentationskultur zu erhöhen, aus der sie leicht gewonnen werden kann.

Die genetische Modifikation der erfindungsgemäßen SES-Moleküle kann auch zu einer indirekten Modulation der Produktion einer oder mehrerer Feinchemikalien führen. Beispielsweise kann man durch Erhöhen der Anzahl oder Aktivität eines erfindungsgemäßen DNA-Reparatur- oder -Rekombinationsproteins die Fähigkeit der Zelle, eine DNA-Schädigung zu entdecken und zu reparieren, erhöhen. Dies sollte die Fähigkeit der Zelle, ein mutiertes Gen in ihrem Genom zu halten, wirksam erhöhen und dadurch die Wahrscheinlichkeit erhöhen, daß ein gentechnologisch in C. *glutami*cum eingebrachtes Transgen (das z.B. ein Protein codiert, das die Biosynthese einer Feinchemikalie steigert) nicht während der Züchtung des Mikroorganismus verloren geht. Dagegen kann es durch Verringern der Anzahl oder Aktivität eines oder mehrerer DNA-Reparatur- oder -Rekombinationsproteine möglich sein, die genetische Instabilität des Organismus zu steigern. Diese Manipulationen sollten die Fähigkeit des Organismus, durch Mutagenese modifiziert zu werden, verbessern, ohne daß die eingebrachte Mutation berichtigt wird. Das gleiche gilt für Proteine, die an der Transposition oder Umlagerung genetischer Elemente in C. glutamicum beteiligt sind (z.B. Transposons). Durch Mutagenese dieser Proteine, so daß ihre Anzahl oder Aktivität entweder gesteigert oder verringert wird, ist es möglich, gleichzeitig die genetische Stabilität des Mikroorganismus zu steigern oder zu verringern. Dies hat eine bedeutende Auswirkung darauf, daß eine andere Mutation in C. *glutamicum* eingebracht und die eingebrachte Mutation beibehalten werden kann. Transposons bieten ebenfalls einen geeigneten Mechanismus, durch den die Mutagenese von *C. glutamicum* durchgeführt werden kann; die Duplikation gewünschter Gene (z.B. von Feinchemikalienbiosynthese-Genen) läßt sich leicht mittels Transposonmutagenese durchführen, wie auch die Disruption ungewünschter Gene (z.B. Gene, die am Abbau gewünschter Feinchemikalien beteiligt sind).

Durch die Modulation eines oder mehrerer Proteine (z.B. Sigma-Faktoren), die an der Regulation der Transkription oder Translation in Reaktion auf besondere Umweltbedingungen beteiligt sind, kann es möglich sein, die Zelle daran zu hindern, daß sie die Proteinsynthese unter ungünstigen Umweltbedingungen, wie man sie in einer Fermenterkultur im Großmaßstab antrifft, verlangsamt oder beendet. Dies sollte zu erhöhter Genexpression führen, was wiederum die gesteigerte Biosynthese gewünschter Feinchemikalien unter diesen Bedingungen ermöglichen kann. Viele dieser sezernierten Proteine haben Funktionen, die für die Zellebensfähigkeit wichtig sind (z.B. Zelloberflächenproteasen oder - Rezeptoren). Eine Änderung des Sekretionswegs, so daß diese Proteine leichter an ihren extrazellulären Ort transportiert werden, kann die Gesamtlebensfähigkeit der Zelle erhöhen und somit zu höheren Zahlen an *C. glutamicum*-Zellen führen, die Feinchemikalien während eine Züchtung im Großmaßstab produzieren können. Da ferner bestimmte bakterielle Proteinsekretionswege (z.B. das sec-System) bekanntlich auch an der Insertion von integralen Membranproteinen (z.B. Rezeptoren, Kanälen, Poren oder Transportern) in die Membran beteiligt sind, kann die Modulation der Aktivität von Proteinen, die an der Proteinsekretion aus C. glutamicum beteiligt sind, die Fähigkeit der Zelle zur Ausscheidung von Abfallprodukten oder zum Import notwendiger von Abfallprodukten oder zum Import notwendiger Metabolite beeinflussen. Ist die Aktivität dieser sekretorischen Proteine erhöht, kann ebenfalls die Fähigkeit der Zelle zur Produktion von Feinchemikalien (durch ein gesteigertes Vorliegen von Transportern/Kanälen in der Membran, die Nährstoffe importieren oder Abfallprodukte ausscheiden können) erhöht sein. Ist die Aktivität dieser sekretorischen Proteine verringert, können nicht genügend Nährstoffe zur Unterstützung der Überproduktion gewünschter Verbindungen vorhanden sein, oder Abfallprodukte können diese Biosynthese stören.

Als Ausgangspunkt zur Herstellung der erfindungsgemäßen Nukleinsäuresequenzen eignet sich das Genom eines *Corynebacterium glutamicum*-Stammes, der von der American Type Culture Collection unter der Bezeichnung ATCC 13032 erhältlich ist.

Von diesen Nukleinsäuresequenzen lassen sich durch die in Tabelle 1 bezeichneten Veränderungen die erfindungsgemäßen Nukleinsäuresequenzen mit üblichen Verfahren herstellen.

Das erfindungsgemäße SES-Protein oder ein biologisch aktiver Abschnitt oder Fragment davon kann an der Reparatur oder Rekombination von DNA, Transposition von genetischem Material, Genexpression (d. h. Transkriptions- oder Translationsprozessen), Proteinfaltung oder Proteinsekretion in *Corynebacterium glutami*cum teilnehmen oder eine oder mehrere der in Tabelle 1 beschriebenen Aktivitäten haben.

In den nachstehenden Unterabschnitten sind verschiedene Aspekte der Erfindung ausführlicher beschrieben:

### A. Isolierte Nukleinsäuremoleküle

Ein Aspekt der Erfindung betrifft isolierte Nukleinsäuremoleküle, die SES-Polypeptide oder biologisch aktive Abschnitte davon codieren, sowie Nukleinsäurefragmente, die zur Verwendung als Hybridisierungssonden oder Primer zur Identifizierung oder Amplifizierung von SES-codierenden Nukleinsäuren (z.B. SES-DNA) hinreichen. Der Begriff "Nukleinsäuremolekül", wie hier verwendet, soll DNA-Moleküle (z.B. cDNA oder genomische DNA) und RNA-Moleküle (z.B. mRNA) sowie DNA- oder RNA-Analoga, die mittels Nukleotidanaloga erzeugt werden, umfassen. Dieser Begriff umfaßt zudem die am 3'- und am 5'-Ende des codierenden Genbereichs gelegene untranslatierte Sequenz: mindestens etwa 100 Nukleotide der Sequenz stromaufwärts des 5'-Endes des codierenden Bereichs und mindestens etwa 20 Nukleotide der Sequenz stromabwärts des 3'-Endes des codierenden Bereichs des Gens. Das Nukleinsäuremolekül kann einzelsträngig oder doppelsträngig sein, ist aber vorzugsweise doppelsträngige DNA. Ein "isoliertes" Nukleinsäuremolekül wird von anderen Nukleinsäuremolekülen abgetrennt, die in der natürlichen Quelle der Nukleinsäure zugegen sind. Eine "isolierte" Nukleinsäure hat vorzugsweise keine Sequenzen, die die Nukleinsäure in der genomischen DNA des Organismus, aus dem die Nukleinsäure stammt, natürlicherweise flankieren (bspw. Sequenzen, die sich am 5'- bzw. 3'-Ende der Nukleinsäure befinden). In verschiedenen Ausführungsformen kann bspw. das isolierte SES-Nukleinsäuremolekül weniger als etwa 5 kb, 4 kb, 3 kb, 2 kb, 1 kb, 0,5 kb oder 0,1 kb der Nukleotidsequenzen, die natürlicherweise das Nukleinsäuremolekül in der genomischen DNA der Zelle, aus der die Nukleinsäure stammt (bspw. eine *C. glutamicum*-Zelle) flankieren. Ein "isoliertes" Nukleinsäuremolekül, wie ein cDNA-Molekül, kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül, bspw. eine Nukleinsäuremolekül mit einer Nukleotidsequenz aus Anhang A oder ein Abschnitt davon, kann mittels molekularbiologischer Standard-Techniken und der hier bereitgestellten Sequenzinformation hergestellt werden. Bspw. kann eine *C. glutamicum*-SES-cDNA aus einer *C. glutamicum*-Bank isoliert werden, indem eine vollständige Sequenz aus Anhang A oder ein Abschnitt davon als Hybridisierungssonde und Standard-Hybridisierungstechniken (wie bspw. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies läßt sich ein Nukleinsäuremolekül, umfassend eine vollständige Sequenz aus Anhang A oder einen Abschnitt davon, durch Polymerasekettenreaktion isolieren, wobei Oligonukleotidprimer, die auf der Basis dieser Sequenz erstellt wurden, verwendet werden (z.B. kann ein Nukleinsäuremolekül, umfassend eine vollständige Sequenz aus Anhang A oder einen Abschnitt davon, durch Polymerasekettenreaktion isoliert werden, indem Oligonukleotidprimer verwendet werden, die auf der Basis dieser gleichen Sequenz aus Anhang A erstellt worden sind). Bspw. läßt sich mRNA aus normalen Endothelzellen isolieren (bspw. durch das Guanidiniumthiocyanat-Extraktionsverfahren von Chirgwin et al. (1979) Biochemistry 18:5294-5299), und die cDNA kann mittels reverser Transkriptase (bspw. Moloney-MLV-Reverse-Transkriptase, erhältlich bei Gibco/BRL, Bethesda, MD, oder AMV-Reverse-Transkriptase, erhältlich von Seikagaku America, Inc., St. Petersburg, FL) hergestellt werden. Synthetische Oligonukleotidprimer für die Amplifizierung via Polymerasekettenreaktion lassen sich auf der Basis einer der in Anhang A gezeigten Nukleotidsequenzen erstellen. Eine erfindungsgemäße Nukleinsäure kann mittels cDNA oder alternativ genomischer DNA als Matrize und geeigneten Oligonukleotidprimern gemäß PCR-Standard-Amplifikationstechniken amplifiziert werden. Die so amplifizierte Nukleinsäure kann in einen geeigneten Vektor kloniert werden und durch DNA-Sequenzanalyse charakterisiert werden. Oligonukleotide, die einer SES-Nukleotidsequenz entsprechen, können ferner durch Standard-Syntheseverfahren, bspw. mit einem automatischen DNA-Synthesegerät, hergestellt werden.

Bei einer bevorzugten Ausführungsform umfaßt ein erfindungsgemäßes isoliertes Nukleinsäuremolekül eine der in Anhang A aufgeführten Nukleotidsequenzen.

Bei einer weiteren bevorzugten Ausführungsform umfaßt ein erfindungsgemäßes isoliertes Nukleinsäuremolekül ein zu einer der in Anhang A gezeigten Nukleotidsequenzen komplementäres Nukleinsäuremolekül oder einen Abschnitt davon, wobei es sich um ein Nukleinsäuremolekül handelt, das zu einer der in Anhang A gezeigten Nukleotidsequenzen hinreichend komplementär ist, daß es mit einer der in Anhang A angegebenen Sequenzen hybridisieren kann, wodurch ein stabiler Duplex entsteht.

Bei einer Ausführungsform codiert das erfindungsgemäße Nukleinsäuremolekül ein Protein oder einen Abschnitt davon, der eine Aminosäuresequenz umfaßt, die hinreichend homolog zu einer Aminosäuresequenz von Anhang B ist, daß das Protein oder ein Abschnitt davon die Fähigkeit behält, an der Reparatur oder Rekombination von DNA, Transposition von genetischem Material, Genexpression (d. h. Transkriptions- oder Translationsprozessen), Proteinfaltung oder Proteinsekretion in *Corynebacterium glutami*cum teilzunehmen. Wie hier verwendet, betrifft der Begriff "hinreichend homolog" Proteine oder Abschnitte davon, deren Aminosäuresequenzen eine minimale Anzahl identischer oder äquivalenter (bspw. einen Aminosäurerest mit einer ähnlichen Seitenkette wie ein Aminosäurerest in einer der Sequenzen von Anhang B) Aminosäurereste zu einer Aminosäuresequenz aus Anhang B aufweisen, so daß das Protein oder ein Abschnitt davon an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teilnehmen kann. An der genetischen Stabilität, Genexpression, Proteinfaltung oder Proteinsekretion von C. *glutamicum* beteiligte Proteine, wie hier beschrieben, können eine Rolle bei der Produktion und Sekretion einer oder mehrerer Feinchemikalien spielen. Beispiele dieser Aktivitäten sind ebenfalls hier beschrieben. Somit trägt die "Funktion eines SES-Proteins" direkt oder indirekt zur Ausbeute, Produktion und/oder Effizienz der Produktion einer oder mehrerer Feinchemikalien bei. Beispiele für SES-Proteine sind in Tabelle 1 gezeigt.

Abschnitte von Proteinen, die von den erfindungsgemäßen SES-Nukleinsäuremolekülen codiert werden, sind vorzugsweise biologisch aktive Abschnitte von einem der SES-Proteine. Der Begriff "biologisch aktiver Abschnitt eines SES-Proteins", wie er hier verwendet wird, soll einen Abschnitt, bspw. eine Domäne/ein Motiv eines SES-Proteins, umfassen, die/das an der Reparatur oder Rekombination von DNA, Transposition von genetischem Material, Genexpression (d. h. Transkriptions- oder Translationsprozessen), Proteinfaltung oder Proteinsekretion in *Corynebacterium* glutamicum teilnimmt oder eine der in Tabelle 1 dargestellten Aktivitäten hat. Zur Bestimmung, ob ein SES-Protein oder ein biologisch aktiver Abschnitt davon an der Reparatur oder Rekombination von DNA, Transposition von genetischem Material, Genexpression (d. h. Transkriptions- oder Translationsprozessen), Proteinfaltung oder Proteinsekretion in *Corynebacterium glutami*cum teilnehmen kann, kann ein Test der enzymatischen Aktivität durchgeführt werden. Diese Testverfahren, wie eingehend in Beispiel 8 des Beispielteils beschrieben, sind dem Fachmann geläufig.

Zusätzlich zu natürlich vorkommenden Varianten der SES-Sequenz, die in der Population existieren können, ist der Fachmann sich ebenfalls bewußt darüber, daß Änderungen durch Mutation in eine Nukleotidsequenz von Anhang A eingebracht werden können, was zur Änderung der Aminosäuresequenz des codierten SES-Proteins führt, ohne daß die Funktionsfähigkeit des SES-Proteins beeinträchtigt wird. Bspw. lassen sich Nukleotidsusbtitutionen, die an "nicht-essentiellen" Aminosäureresten zu Aminosäuresubstitutionen führen, in einer Sequenz von Anhang A herstellen. Ein "nichtessentieller" Aminosäurerest ist ein Rest, der sich in der Wildtypsequenz von einem der SES-Proteine (Anhang B) verändern läßt, ohne daß die Aktivität des SES-Proteins verändert wird, wohingegen ein "essentieller" Aminosäurerest für die SES-Proteinaktivität erforderlich ist. Andere Aminosäurereste jedoch (bspw. nicht-konservierte oder lediglich semikonservierte Aminosäurereste in der Domäne mit SES-Aktivität) können für die Aktivität nicht essentiell sein und lassen sich somit wahrscheinlich verändern, ohne daß die SES-Aktivität verändert wird.

Ein isoliertes Nukleinsäuremolekül, das ein SES-Protein codiert, das zu einer Proteinsequenz aus Anhang B homolog ist, kann durch Einbringen von einer oder mehreren Nukleotidsubstitutionen, - additionen oder -deletionen in eine Nukleotidsequenz aus Anhang A erzeugt werden, so daß eine oder mehrere Aminosäuresubstitutionen, -additionen oder -deletionen in das codierte Protein eingebracht werden. Die Mutationen können in eine der Sequenzen aus Anhang A durch Standard-Techniken, wie stellengerichtete Mutagenese und PCR-vermittelte Mutagenese, eingebracht werden. Vorzugsweise werden konservative Aminosäuresubstitutionen an einem oder mehreren der vorhergesagten nicht-essentiellen Aminosäurereste eingeführt. Bei einer "konservativen Aminosäuresubstitution" wird der Aminosäurerest durch einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), nicht-polaren Seitenketten, (bspw. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nichtessentieller Aminosäurerest in einem SES-Protein wird somit vorzugsweise durch einen anderen Aminosäurerest der gleichen Seitenkettenfamilie ausgetauscht. In einer weiteren Ausführungsform können die Mutationen alternativ zufallsgemäß über die gesamte oder einen Teil der SES-codierenden Sequenz eingebracht werden, bspw. durch Sättigungsmutagenese, und die resultierenden Mutanten können auf eine hier beschriebene SES-Aktivität untersucht werden, um Mutanten zu identifizieren, die eine SES-Aktivität beibehalten. Nach der Mutagenese von einer der Sequenzen aus Anhang A kann das codierte Protein rekombinant exprimiert werden, und die Aktivität des Proteins kann bspw. mit den hier beschriebenen Tests (siehe Beispiel 8 des Beispielteils) bestimmt werden.

### B. Rekombinante Expressionsvektoren und Wirtszellen

Ein weiterer Aspekt der Erfindung betrifft Vektoren, vorzugsweise Expressionsvektoren, die eine Nukleinsäure enthalten, die ein SES-Protein (oder einen Abschnitt davon) codieren. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzliche DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (bspw. Bakterienvektoren mit bakteriellem Replikationsursprung und episomale Säugetiervektoren). Andere Vektoren (z.B. nicht-episomale Säugetiervektoren) werden in das Genom einer Wirtszelle beim Einbringen in die Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben die Expressionsvektoren, die bei DNA-Rekombinationstechniken verwendet werden können, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch andere Expressionsvektorformen, wie virale Vektoren (bspw. replikationsdefiziente Retroviren, Adenoviren und adenoverwandte Viren), die ähnliche Funktionen ausüben, umfassen.

Die erfindungsgemäßen rekombinanten Expressionsvektoren umfassen eine erfindungsgemäße Nukleinsäure in einer Form, die sich zur Expression der Nukleinsäure in einer Wirtszelle eignet, d.h. daß die rekombinanten Expressionsvektoren eine oder mehrere regulatorische Sequenzen, ausgewählt auf der Basis der zur Expression zu verwendenden Wirtszellen, umfassen, die mit der zu exprimierenden Nukleinsäuresequenz funktionsfähig verbunden sind. In einem rekombinanten Expressionsvektor bedeutet "funktionsfähig verbunden", daß die Nukleotidsequenz von Interesse derart an die regulatorische(n) Sequenz(en) gebunden ist, daß die Expression der Nukleotidsequenz möglich ist (bspw. in einem in-vitro-Transkriptions-/Translationssystem oder in einer Wirtszelle, wenn der Vektor in die Wirtszelle eingebracht ist). Der Begriff "regulatorische Sequenz" soll Promotoren, Enhancer und andere Expressionskontrollelemente (bspw. Polyadenylierungssignale) umfassen. Diese regulatorischen Sequenzen sind bspw beschrieben in Goeddel: Gene Expression Technology: Methods in Enzymology 185, Academic Press, San Diego, CA (1990). Regulatorische Sequenzen umfassen solche, die die konstitutive Expression einer Nukleotidsequenz in vielen Wirtszelltypen steuern, und solche, die die Expression der Nukleotidsequenz nur in bestimmten Wirtszellen steuern. Der Fachmann ist sich dessen bewußt, daß die Gestaltung eines Expressionsvektors von Faktoren abhängen kann, wie der Wahl der zu transformierenden Wirtszelle, dem gewünschten Ausmaß der Proteinexpression usw. Die erfindungsgemäßen Expressionsvektoren können in die Wirtszellen eingebracht werden, so daß dadurch Proteine oder Peptide, einschließlich der Fusionsproteine oder -peptide, die von den Nukleinsäuren, wie hier beschrieben, codiert werden, hergestellt werden (bspw. SES-Proteine, mutierte Formen von SES-Proteinen, Fusionsproteine, usw.).

Die erfindungsgemäßen rekombinanten Expressionsvektoren können zur Expression von SES-Proteinen in prokaryotischen oder eukaryotischen Zellen ausgestaltet sein. Bspw. können SES-Gene in bakteriellen Zellen, wie C. *glutamicum,* Insektenzellen (mit Baculovirus-Expressionsvektoren), Hefe- und anderen Pilzzellen (siehe Romanos, M.A. et al. (1992) "Foreign gene expression in yeast: a review", Yeast 8: 423-488; van den Hondel, C.A.M.J.J. et al. (1991) "Heterologous gene expression in filamentous fungi" in: More Gene Manipulations in Fungi, J.W. Bennet & L.L. Lasure, Hrsg., S. 396-428: Academic Press: San Diego; und van den Hondel, C.A.M.J.J. & Punt, P.J. (1991) "Gene transfer systems and vector development for filamentous fungi. in: Applied Molecular Genetics of Fungi, Peberdy, J.F. et al., Hrsg, S. 1-28, Cambridge University Press: Cambridge), Algenzellen und Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.: 583-586) oder Säugetierzellen exprimiert werden. Geeignete Wirtszellen werden weiter erörtert in Goeddel, Gene Expression Technology: Methods in Enzymology 185, Academic Press, San Diego, CA (1990). Der rekombinante Expressionsvektor kann alternativ, bspw. mit regulatorischen Sequenzen des T7-Promotors und T7-Polymerase, in vitro transkribiert und translatiert werden.

Die Expression von Proteinen in Prokaryoten erfolgt meist mit Vektoren, die konstitutive oder induzierbare Promotoren enthalten, die die Expression von Fusions- oder Nicht-Fusionsproteinen steuern. Fusionsvektoren steuern eine Reihe von Aminosäuren zu einem darin codierten Protein, gewöhnlich am Aminoterminus des rekombinanten Proteins, bei. Diese Fusionsvektoren haben gewöhnlich drei Aufgaben: 1) die Verstärkung der Expression von rekombinantem Protein; 2) die Erhöhung der Löslichkeit des rekombinanten Proteins; und 3) die Unterstützung der Reinigung des rekombinanten Proteins durch Wirkung als Ligand bei der Affinitätsreinigung. Bei Fusions-Expressionsvektoren wird oft eine proteolytische Spaltstelle an der Verbindungsstelle der Fusionseinheit und des rekombinanten Proteins eingebracht, so daß die Trennung des rekombinanten Proteins von der Fusionseinheit nach der Reinigung des Fusionsproteins möglich ist. Diese Enzyme und ihre entsprechenden Erkennungssequenzen umfassen Faktor Xa, Thrombin und Enterokinase.

Übliche Fusionsexpressionsvektoren umfassen pGEX (Pharmacia Biotech Inc; Smith, D.B. und Johnson, K.S. (1988) Gene 67:31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird. Bei einer Ausführungsform ist die codierende Sequenz des SES-Proteins in einen pGEX-Expressionsvektor kloniert, so daß ein Vektor erzeugt wird, der ein Fusionsprotein codiert, umfassend vom N-Terminus zum C-Terminus: GST - Thrombin-Spaltstelle - X-Protein. Das Fusionsprotein kann durch Affinitätschromatographie mittels Glutathion-Agarose-Harz gereinigt werden. Das rekombinante SES-Protein, das nicht mit GST fusioniert ist, kann durch Spaltung des Fusionsproteins mit Thrombin gewonnen werden.

Beispiele geeigneter induzierbarer Nicht-Fusions-E.-coli-Expressionsvektoren umfassen pTrc (Amann et al., (1988) Gene 69:301-315) und pET 11d (Studier et al. Gene Expression Technology: Methods in Enzymology 185, Academic Press, San Diego, Kalifornien (1990) 60-89). Die Zielgenexpression aus dem pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL 21 (DE3) oder HMS174 (DE3) von einem residenten λ-Prophagen geliefert, der ein T7 gnl-Gen unter der Transkriptionskontrolle des lacUV 5-Promotors birgt.

Eine Strategie zur Maximierung der Expression des rekombinanten Proteins ist die Expression des Proteins in einem Wirtsbakterium, dessen Fähigkeit zur proteolytischen Spaltung des rekombinanten Proteins gestört ist (Gottesman, S. Gene Expression Technology: Methods in Enzymology 185, Academic Press, San Diego, Kalifornien (1990) 119-128). Eine weitere Strategie ist die Veränderung der Nukleinsäuresequenz der in einen Expressionsvektor zu inserierenden Nukleinsäure, so daß die einzelnen Codons für jede Aminosäure diejenigen sind, die vorzugsweise in einem zur Expression ausgewählten Bakterium, wie C. *glutamicum,* verwendet werden (Wada et al. (1992) Nucleic Acids Res. 20:2111-2118). Diese Veränderung der erfindungsgemäßen Nukleinsäuresequenzen kann durch Standard-DNA-Synthesetechniken erfolgen.

Bei einer weiteren Ausführungsform ist der SES-Protein-Expressionsvektor ein Hefe-Expressionsvektor. Beispiele für Vektoren zur Expression in der Hefe *S. cerevisiae* umfassen pYepSec1 (Baldari et al., (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of Fungi, J.F. Peberdy et al., Hrsg., S. 1-28, Cambridge University Press: Cambridge.

Alternativ können die erfindungsgemäßen SES-Proteine in Insektenzellen unter Verwendung von Baculovirus-Expressionsvektoren exprimiert werden. Baculovirus-Vektoren, die zur Expression von Proteinen in gezüchteten Insektenzellen (bspw. Sf9-Zellen) verfügbar sind, umfassen die pAc-Reihe (Smith et al., (1983) Mol. Cell Biol.. 3:2156-2165) und die pVL-Reihe (Lucklow und Summers (1989) Virology 170:31-39).

In einer weiteren Ausführungsform können die erfindungsgemäßen SES-Proteine in Zellen einzelliger Pflanzen (wie Algen) oder in Pflanzenzellen höherer Pflanzen (bspw. Spermatophyten, wie Feldfrüchte) exprimiert werden. Beispiele für Pflanzen-Expressionsvektoren umfassen solche, die eingehend beschrieben sind in: Becker, D., Kemper, E., Schell, J. und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721.

In einer weiteren Ausführungsform wird eine erfindungsgemäße Nukleinsäure in Säugetierzellen mit einem Säugetier-Expressionsvektor exprimiert. Beispiele für Säugetier-Expressionsvektoren umfassen pCDM8 (Seed, B. (1987) Nature 329:840) und pMT2PC (Kaufman et al. (1987) EMBO J. 6:187-195). Bei der Verwendung in Säugetierzellen werden die Kontrollfunktionen des Expressionsvektors oft von viralen regulatorischen Elementen bereitgestellt. Gemeinhin verwendete Promotoren stammen bspw. aus Polyoma, Adenovirus 2, Cytomegalievirus und Simian Virus 40. Weitere geeignete Expressionssysteme für prokaryotische und eukaryotische Zellen siehe in Kapitel 16 und 17 von Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989.

Bei einer weiteren Ausführungsform kann der rekombinante Säugetier-Expressionsvektor die Expression der Nukleinsäure vorzugsweise in einem bestimmten Zelltyp bewirken (bspw. werden gewebespezifische regulatorische Elemente zur Expression der Nukleinsäure verwendet). Gewebespezifische regulatorische Elemente sind im Fachgebiet bekannt. Nicht-einschränkende Beispiele für geeignete gewebespezifische Promotoren umfassen den Albuminpromotor (leberspezifisch; Pinkert et al. (1987) Genes Dev. 1:268-277), lymphoid-spezifische Promotoren (Calame und Eaton (1988) Adv. Immunol. 43:235-275), insbesondere Promotoren von T-Zellrezeptoren (Winoto und Baltimore (1989) EMBO J. 8:729-733) und Immunglobulinen (Banerji et al. (1983) Cell 33:729-740; Queen und Baltimore (1983) Cell 33:741-748), neuronenspezifische Promotoren (bspw. der Neurofilament-Promotor; Byrne und Ruddle (1989) PNAS 86:5473-5477), pankreasspezifische Promotoren (Edlund et al., (1985) Science 230:912-916) und milchdrüsenspezifische Promotoren (bspw. Milchserum-Promotor; US-Patent Nr. 4 873 316 und europäische Patentanmeldungsveröffentlichung Nr. 264 166). Entwicklungsregulierte Promotoren sind ebenfalls umfaßt, bspw. die Maus-hox-Promotoren (Kessel und Gruss (1990) Science 249:374-379) und der α-Fetoprotein-Promotor (Campes und Tilghman (1989) Genes Dev. 3:537-546).

Die Erfindung stellt zudem einen rekombinanten Expressionsvektor bereit, umfassend ein erfindungsgemäßes DNA Molekül, das in Antisense-Richtung in den Expressionsvektor kloniert ist. D.h. daß das DNA-Molekül derart mit einer regulatorischen Sequenz funktionsfähig verbunden ist, daß die Expression (durch Transkription des DNA-Moleküls) eines RNA-Moleküls, das zur SES-mRNA antisense ist, möglich wird. Es können regulatorische Sequenzen ausgewählt werden, die funktionsfähig an eine in Antisense-Richtung klonierte Nukleinsäure gebunden sind und die kontinuierliche Expression des Antisense-RNA-Moleküls in einer Vielzahl von Zelltypen steuern, bspw. können virale Promotoren und/oder Enhancer oder regulatorische Sequenzen ausgewählt werden, die die konstitutive, gewebespezifische oder zelltypspezifische Expression von Antisense-RNA steuern. Der Antisense-Expressionsvektor kann in Form eines rekombinanten Plasmids, Phagemids oder attenuierten Virus vorliegen, in dem Antisense-Nukleinsäuren unter der Kontrolle eines hochwirksamen regulatorischen Bereichs produziert werden, dessen Aktivität durch den Zelltyp bestimmt wird, in den der Vektor eingebracht wird. Für eine Diskussion der Regulation der Genexpression mittels Antisense-Genen siehe Weintraub, H. et al., Antisense-RNA as a molecular tool for genetic analysis, Reviews - Trends in Genetics, Bd. 1(1) (1986).

Ein weiterer Aspekt der Erfindung betrifft Wirtszellen, in die ein erfindungsgemäßer rekombinanter Expressionsvektor eingebracht worden ist. Die Begriffe "Wirtszelle" und "rekombinante Wirtszelle" werden hier untereinander austauschbar verwendet. Es ist selbstverständlich, daß diese Begriffe nicht nur eine bestimmte Zielzelle, sondern auch die Nachkommen oder potentiellen Nachkommen dieser Zelle betreffen. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch im Umfang des Begriffs, wie er hier verwendet wird, noch umfaßt.

Eine Wirtszelle kann eine prokaryotische oder eukaryotische Zelle sein. Bspw. kann ein SES-Protein in Bakterienzellen, wie C. glutamicum, Insektenzellen, Hefe- oder Säugetierzellen (wie O-varzellen des chinesischen Hamsters (CHO) oder COS-Zellen) exprimiert werden. Andere geeignete Wirtszellen sind dem Fachmann geläufig. Mikroorganismen, die mit *Corynebacterium glutami*cum verwandt sind und sich geeignet als Wirtszellen für die erfindungsgemäßen Nukleinsäure- und Proteinmoleküle verwenden lassen, sind in Tabelle 3 aufgeführt.

Durch herkömmliche Transformations- oder Transfektionsverfahren läßt sich Vektor-DNA in prokaryotische oder eukaryotische Zellen einbringen. Die Begriffe "Transformation" und "Transfektion", "Konjugation" und "Transduktion", wie sie hier verwendet werden, sollen eine Vielzahl von im Stand der Technik bekannten Verfahren zum Einbringen fremder Nukleinsäure (bspw. DNA) in eine Wirtszelle umfassen, einschließlich Calciumphosphat- oder Calciumchlorid-Copräzipitation, DEAE-Dextran-vermittelter Transfektion, Lipofektion, natürlicher Kompetenz, chemisch vermittelter Übertragung oder Elektroporation. Geeignete Verfahren zur Transformation oder Transfektion von Wirtszellen lassen sich nachlesen in Sambrook et al. (Molecular Cloning: A Laboratory Manual. 2. Aufl., Cold Spring Harbor Laboratory, Cold Spring Harbor Laboratory Press, Cold Spring Harbor, NY, 1989) und anderen Labor-Handbüchern.

Es ist bekannt, daß für die stabile Transfektion von Säugetierzellen je nach dem verwendeten Expressionsvektor und der verwendeten Transfektionstechnik nur ein kleiner Teil der Zellen die fremde DNA in ihr Genom integrieren kann. Zur Identifizierung und Selektion dieser Integranten wird gewöhnlich ein Gen, das einen selektierbaren Marker (z.B. Resistenz gegen Antibiotika) codiert, zusammen mit dem Gen von Interesse in die Wirtszellen eingebracht. Bevorzugte selektierbare Marker umfassen solche, die die Resistenz gegen Medikamente, wie G418, Hygromycin und Methotrexat, verleihen. Eine Nukleinsäure, die einen selektierbaren Marker codiert, kann in eine Wirtszelle auf dem gleichen Vektor eingebracht werden, wie derjenige, der ein SES-Protein codiert, oder kann auf einem gesonderten Vektor eingebracht werden. Zellen, die mit der eingebrachten Nukleinsäure stabil transfiziert worden sind, können bspw. durch Medikamentenselektion identifiziert werden (z.B. überleben Zellen, die den selektierbaren Marker integriert haben, wohingegen die anderen Zellen sterben).

Zur Erzeugung eines homolog rekombinierten Mikroorganismus wird ein Vektor hergestellt, der zumindest einen Abschnitt eines SES-Gens enthält, in den eine Deletion, Addition oder Substitution eingebracht worden ist, um das SES-Gen zu verändern, bspw. funktionell zu disrumpieren. Dieses SES-Gen ist vorzugsweise ein *Corynebacterium* glutamicum-SES-Gen, jedoch kann ein Homologon von einem verwandten Bakterium oder sogar aus einer Säugetier-, Hefe- oder Insektenquelle verwendet werden. Bei einer bevorzugten Ausführungsform ist der Vektor derart ausgestaltet, daß das endogene SES-Gen bei homologer Rekombination funktionell disrumpiert ist (d.h. nicht länger ein funktionelles Protein codiert; auch als "Knockout"-Vektor bezeichnet). Der Vektor kann alternativ derart ausgestaltet sein, daß das endogene SES-Gen bei homologer Rekombination mutiert oder anderweitig verändert ist, jedoch noch das funktionelle Protein codiert (z.B. kann der stromaufwärts gelegene regulatorische Bereich derart verändert sein, daß dadurch die Expression des endogenen SES-Proteins verändert wird.). Der veränderte Abschnitt des SES-Gens ist im homologen Rekombinationsvektor an seinem 5'- und 3'-Ende von zusätzlicher Nukleinsäure des SES-Gens flankiert, die eine homologe Rekombination zwischen dem exogenen SES-Gen, das von dem Vektor getragen wird, und einem endogenen SES-Gen in einem Mikroorganismus ermöglicht. Die zusätzliche flankierende SES-Nukleinsäure ist für eine erfolgreiche homologe Rekombination mit dem endogenen Gen hinreichend lang. Gewöhnlich enthält der Vektor mehrere Kilobasen flankierende DNA (sowohl am 5'- als auch am 3'-Ende) (siehe z.B. Thomas, K.R. und Capecchi, M.R. (1987) Cell 51:503 für eine Beschreibung von homologen Rekombinationsvektoren). Der Vektor wird in einen Mikroorganismus (z.B. durch Elektroporation) eingebracht, und Zellen, in denen das eingebrachte SES-Gen mit dem endogenen SES-Gen homolog rekombiniert ist, werden unter Verwendung im Fachgebiet bekannter Verfahren selektiert.

Bei einer anderen Ausführungsform können rekombinante Mikroorganismen produziert werden, die ausgewählte Systeme enthalten, die eine regulierte Expression des eingebrachten Gens ermöglichen. Der Einschluß eines SES-Gens in einen Vektor, wodurch es unter die Kontrolle des Lac-Operons gebracht wird, ermöglicht z.B. die Expression des SES-Gens nur in Gegenwart von IPTG. Diese regulatorischen Systeme sind im Fachgebiet bekannt.

Eine erfindungsgemäße Wirtszelle, wie eine prokaryotische oder eukaryotische Wirtszelle in Kultur, kann zur Produktion (d.h. Expression) eines SES-Proteins verwendet werden. Die Erfindung stellt zudem Verfahren zur Produktion von SES-Proteinen unter Verwendung der erfindungsgemäßen Wirtszellen bereit. Bei einer Ausführungsform umfaßt das Verfahren die Anzucht der erfindungsgemäßen Wirtszelle (in die ein rekombinanter Expressionsvektor, der ein SES-Protein codiert, eingebracht worden ist, oder in deren Genom ein Gen eingebracht worden ist, das ein Wildtyp-oder verändertes SES-Protein codiert) in einem geeigneten Medium, bis das SES-Protein produziert worden ist. Das Verfahren umfaßt in einer weiteren Ausführungsform das Isolieren der SES-Proteine aus dem Medium oder der Wirtszelle.

### C. Erfindungsgemäße Verwendungen und Verfahren

Die hier beschriebenen Nukleinsäuremoleküle, Proteine, Proteinhomologa, Fusionsproteine, Primer, Vektoren und Wirtszellen können in einem oder mehreren nachstehenden Verfahren verwendet werden: Identifikation von C. *glutamicum* und verwandten Organismen, Kartierung von Genomen von Organismen, die mit C. *glutami*cum verwandt sind, Identifikation und Lokalisation von *C. glutamicum*-Sequenzen von Interesse, Evolutionsstudien, Bestimmung von SES-Proteinbereichen, die für die Funktion notwendig sind, Modulation der Aktivität eines SES-Proteins; Modulation des Stoffwechsels einer oder mehrerer Zellmembrankomponenten; Modulation des Transmembrantransports einer oder mehrerer Verbindungen und Modulation der zellulären Produktion einer gewünschten Verbindung, wie einer Feinchemikalie. Die erfindungsgemäßen SES-Nukleinsäuremoleküle haben eine Vielzahl von Verwendungen. Sie können zunächst zur Identifikation eines Organismus als *Corynebacterium glutamicum* oder naher Verwandter davon verwendet werden. Sie können zudem zur Identifikation des Vorliegens von C. *glutamicum* oder eines Verwandten davon in einer Mischpopulation von Mikroorganismen verwendet werden. Die Erfindung stellt die Nukleinsäuresequenzen einer Reihe von *C. glutamicum*-Genen bereit. Durch Sondieren der extrahierten genomischen DNA einer Kultur einer einheitlichen oder gemischten Population von Mikroorganismen unter stringenten Bedingungen mit einer Sonde, die einen Bereich eines C. *glutamicum-Gens* überspannt, das für diesen Organismus einzigartig ist, kann man bestimmen, ob dieser Organismus zugegen ist. *Corynebacterium glutamicum* selbst ist zwar nicht pathogen, jedoch ist es mit pathogenen Arten, wie *Corynebacterium diptheriae*, verwandt. Der Nachweis eines solchen Organismus ist von signifikanter klinischer Bedeutung.

Die erfindungsgemäßen Nukleinsäure- und Proteinmoleküle können ferner als marker für bestimmte Bereiche des Genoms dienen. Dies eignet sich nicht nur zum Kartieren des Genoms, sondern auch für funktionelle Studien von *C. glutamicum*-Proteinen. Zur Identifikation des Genombereichs, an den ein bestimmtes *C. glutamicum-*DNA-bindendes Protein bindet, kann das *C. glutamicum*-Genom bspw. gespalten und die Fragmente mit dem DNA-bindenden Protein inkubiert werden. Diejenigen, die das Protein binden, können zusätzlich mit den erfindungsgemäßen Nukleinsäuremolekülen, vorzugsweise mit leicht nachweisbaren Markierungen, sondiert werden; die Bindung eines solchen Nukleinsäuremoleküls an das Genomfragment ermöglicht die Lokalisation des Fragmentes auf der genomischen Karte von *C. glutamicum*, und wenn dies mehrmals mit unterschiedlichen Enzymen durchgeführt wird, erleichtert es eine rasche Bestimmung der Nukleinsäuresequenz, an die das Protein bindet. Die erfindungsgemäßen Nukleinsäuremoleküle können zudem hinreichend homolog zu den Sequenzen verwandter Arten sein, so daß diese Nukleinsäuremoleküle als Marker zur Konstruktion einer genomischen Karte in verwandten Bakterien (z.B. Brevibacterium *lactofermentum)* dienen können.

Die erfindungsgemäßen SES-Nukleinsäuremoleküle eignen sich ebenfalls für Evolutions- und Proteinstruktur-Untersuchungen. Die Stoffwechsel- und Transportprozesse, an denen die erfindungsgemäßen Moleküle beteiligt sind, werden von einer Vielzahl von prokaryotischen und eukaryotischen Zellen ausgenutzt; durch Vergleich der Sequenzen der erfindungsgemäßen Nukleinsäuremoleküle mit solchen, die ähnliche Enzyme aus anderen Organismen codieren, kann der Evolutions-Verwandschaftsgrad der Organismen bestimmt werden. Entsprechend ermöglicht ein solcher Vergleich die Bestimmung, welche Sequenzbereiche konserviert sind und welche nicht, was bei der Bestimmung solcher Bereiche des Proteins hilfreich sein kann, die für die Enzymfunktion essentiell sind. Dieser Typ der Bestimmung ist für Proteintechnologie-Untersuchungen wertvoll und kann einen Hinweis darauf geben, wieviel Mutagenese das Protein tolerieren kann ohne die Funktion zu verlieren.

Die Manipulation der erfindungsgemäßen SES-Nukleinsäuremoleküle kann die Produktion von SES-Proteinen mit funktionellen Unterschieden zu den Wildtyp-SES-Proteinen bewirken. Diese Proteine können hinsichtlich ihrer Effizienz oder Aktivität verbessert werden, können in größerer Anzahl als gewöhnlich in der Zelle zugegen sein oder können hinsichtlich ihrer Effizienz oder Aktivität geschwächt sein.

Diese Modulation der Aktivität von Proteinen, die an der DNA-Reparatur, Rekombination oder Transposition in C. *glutamicum* beteiligt sind, sollte die genetische Stabilität der Zelle beeinflussen. Beispielsweise kann man durch Verringern der Anzahl oder Aktivität von Proteinen, die an DNA-Reparaturmechanismen beteiligt sind, die Fähigkeit der Zelle, genetische Fehler zu korrigieren, verringern, was das einfachere Einbringen gewünschter Mutationen in das Genom (wie solchen, die an der Feinchemikalienproduktion beteiligte Proteine codieren) ermöglichen sollte. Die Steigerung der Aktivität oder Anzahl von Transposons sollte ebenso zu einer erhöhten Mutationsrate im Genom führen und kann die leichte Verdopplung gewünschter Gene (bspw. solcher, die an der Feinchemikalienproduktion beteiligte Proteine codieren) oder Disruption unerwünschter Gene (z.B. solcher, die Feinchemikalien-Abbauproteine codieren) möglich machen. Dagegen kann es durch Verringern der Anzahl oder Aktivität von Transposons oder Erhöhen der Anzahl oder Aktivität von DNA-Reparaturproteinen möglich sind, die genetische Stabilität von *C. glutamicum* zu erhöhen, was wiederum zur besseren Aufrechterhaltung eingebrachter Mutationen in diesen Mikroorganismen durch mehrere Generationen in Kultur führen sollte. Idealerweise wird während der Mutagenese und Stammkonstruktion die Aktivität eines oder mehrerer DNA-Reparatursysteme verringert und die Aktivität eines oder mehrerer Transposons erhöht, aber wenn die gewünschte Mutation in dem Stamm erzielt worden ist, tritt das Gegenteil auf. Diese Manipulation ist möglich, indem ein oder mehrere DNA-Reparaturgene oder Transposons unter die Kontrolle eines induzierbaren Repressors gestellt wird/werden.

Die Modulation von an der Transkription und Translation in C. *glutamicum* beteiligten Proteinen kann direkte und indirekte Wirkungen auf die Produktion einer Feinchemikalien von diesen Mikroorganismen haben. Beispielsweise ist es durch Manipulation eines Proteins, das direkt ein Gen translatiert (z.B. einer Polymerase) oder direkt die Transkription reguliert (eines Repressor- oder Aktivatorproteins) möglich, die Expression des Zielgens direkt zu beeinflussen. Bei Genen, die ein Protein codieren, das an der Biosynthese oder am Abbau einer Feinchemikalie beteiligt ist sollte dieser Typ der genetischen Manipulation eine direkte Wirkung auf die Produktion dieser Feinchemikalie haben. Die Mutagenese eines Repressorproteins, so daß es sein Zielgen nicht länger reprimieren kann, oder die Mutagenese eines Aktivatorproteins, so daß seine Aktivität optimiert wird, sollte zu einer erhöhten Transkription des Zielgens führen. Ist das Zielgen z.B. ein Feinchemikalienbiosynthesegen, kann eine erhöhte Produktion dieser Chemikalie aufgrund der insgesamt größeren Anzahl an für vorliegenden Transkripten dieses Gens resultieren, was ebenfalls zu einer vergrößerten Anzahl des Proteins führen sollte. Die Erhöhung der Anzahl oder Aktivität eines Repressorproteins für eine Zielsequenz oder die Verringerung der Anzahl oder Aktivität eines Aktivatorproteins für eine Zielsequenz sollte, wenn diese Sequenz bspw. ein Feinchemikalien-Abbauprotein ist, zu einer ähnlichen Steigerung der Produktion der Feinchemikalie führen.

Auch indirekte Wirkungen auf die Feinchemikalienproduktion können aus der Manipulation von Proteinen, die an der Transkription und Translation beteiligt sind, hervorgehen. Durch die Modulation der Aktivität oder Anzahl von Transkriptionsfaktoren (z.B. der Sigma-Faktoren) oder von Translationsrepressoren/aktivatoren, die die Transkription in *C. glutamicum* in Reaktion auf Umwelt- oder Stoffwechselfaktoren global regulieren, sollte es möglich sein, die zelluläre Transkription von der Umwelt-oder Stoffwechselregulation abzukoppeln. Dies könnte wiederum eine kontinuierliche Transkription unter Bedingungen ermöglichen, die gewöhnlich die Genexpression verlangsamen oder beenden würden, wie ungünstigen Bedingungen (z.B. hohe Temperatur, niedriger Sauerstoffgehalt, hoher Spiegel an Abfallprodukten), die in einer Fermenterkultur im Großmaßstab vorliegen. Durch Erhöhen der Rate der Expression des Gens (z.B. Feinchemikalienbiosynthesegens) in diesen Situationen kann auch, zumindest aufgrund der vergleichsweise größeren Anzahl der Feinchemikalienbiosynthese-proteine in der Zelle, die Gesamtrate der Feinproduktproduktion erhöht werden. Prinzipien und Beispiele für die Modifikation der Transkriptions- und Translationsregulation sind beschrieben in z.B. Lewin, B. (1990) Genes IV, Teil 3: "Controlling procaryotic genes by transcription", Oxford Univ. Press: Oxford, S. 213-301.

Die Modulation der Aktivität oder Anzahl von Proteinen, die an der Polypeptidfaltung beteiligt sind (z.B. Chaperone) kann insgesamt die Erhöhung der Produktion korrekt gefalteter Moleküle in der Zelle ermöglichen. Dies hat zwei Auswirkungen: zunächst insgesamt eine Erhöhung der Anzahl an Proteinen in der Zelle aufgrund der Tatsache, daß weniger Proteine falsch gefaltet und abgebaut werden und, zweitens, eine Erhöhung der Anzahl eines gegebenen Proteins, das korrekt gefaltet und somit aktiv ist (s. z.B. Thomas, J.G., Baneyx, F. (1997) Protein Expression and Purification 11(3):289-296; Luo, Z.H., und Hua, Z.C. (1998) Biochemistry and Molecular Biology International 46(3):471-477; Dale, G.E., et al. (1994) Protein Engineering 7(7):925-931; Amrein, K.E. et al. (1995) Proc. Natl. Acad. Sci. U.S.A. 92(4):1048-1052 und Caspers, P., et al. (1994) Cell. Mol. Biol. 40(5):635-644). Solche Mutationen führen zwar zu einer Erhöhung der Anzahl aktiver Proteine jeder Art, aber wenn sie mit zusätzlichen Mutationen, die die Aktivität oder Anzahl von z.B. einem Feinchemikalienbiosyntheseprotein erhöhen, gekoppelt werden, kann eine additive Wirkung auf die Menge an korrekt gefaltetem aktivem gewünschtem Protein erhalten werden.

Die Manipulation von Proteinen, die an der Sekretion von Polypeptiden aus C. *glutamicum* beteiligt sind, so daß ihre Aktivität oder Anzahl verbessert ist, kann die Sekretion einer proteinartigen Feinchemikalie (z.B. eines Enzyms) aus diesem Mikroorganismus direkt verbessern. Es ist erheblich leichter, Feinchemikalien zu ernten und zu reinigen, wenn sie in das Medium einer Kultur im Großmaßstab sezerniert werden als wenn sie in der Zelle zurückgehalten werden, so daß die Ausbeute und Produktion einer Feinchemikalie durch diese Veränderung des Sekretionssystems erhöht werden sollte. Die genetische Manipulationen dieser Sekretionsproteine kann auch zu direkten Verbesserungen der Produktion einer oder mehrerer Feinchemikalien führen. Erstens kann die gesteigerte oder verringerte Aktivität eines oder mehrerer C. glutamicum-Sekretionssysteme (wie sie durch Mutagenese eines oder mehrerer SES-Proteine, die an diesen Wegen beteiligt sind, erzielt wird) zu insgesamt erhöhten oder verringerten Sekretionsraten aus der Zelle führen. Viele dieser sezernierten Proteine haben Funktionen, die für die Zellebensfähigkeit wichtig sind (z.B. Zelloberflächenproteasen oder -Rezeptoren). Eine Änderung des Sekretionswegs, so daß diese Proteine leichter an ihren extrazellulären Ort transportiert werden, kann die Gesamtlebensfähigkeit der Zelle erhöhen und somit zu höheren Zahlen an C. *glu*tamicum-Zellen führen, die Feinchemikalien während eine Züchtung im Großmaßstab produzieren können. Zweitens spielen bestimmte bakterielle Sekretionssysteme (z.B. das sec-System) bekanntlich auch eine signifikante Rolle bei dem Prozeß, durch den integrale Membranproteinen (z.B. Kanäle, Poren oder Transporter) in die Zellmembran inserieren. Wird die Aktivität eines oder mehrerer Sekretionswegproteine gesteigert, kann die Fähigkeit der Zelle, Feinchemikalien zu produzieren, aufgrund des Vorliegens erhöhten intrazellulärer Nährstoffmengen oder verringerter Mengen an intrazellulären Abfallstoffen ebenfalls erhöht werden. Ist die Aktivität eines oder mehrerer dieser Sekretionswegproteine verringert, können nicht genügend Nährstoffe zur Unterstützung der Überproduktion gewünschter Verbindungen vorhanden sein, oder Abfallprodukte können diese Biosynthese stören.

Diese vorstehend genannten Mutagenesestrategien für SES-Proteine, die erhöhte Ausbeuten einer Feinchemikalie aus C. *glutamicum* bewirken sollen, sollen nicht einschränkend sein; Variationen dieser Mutagenesestrategien sind dem Fachmann leicht ersichtlich. Unter Verwendung dieser Strategien und einschließlich der hier offenbarten Mechanismen können die erfindungsgemäßen Nukleinsäure- und Proteinmoleküle verwendet werden, um *C. glutamicum-* oder verwandte Bakterienstämme, die mutierte SES-Nukleinsäure- und Proteinmoleküle exprimieren, zu erzeugen, so daß die Ausbeute, Produktion und/oder Effizienz der Produktion einer gewünschten Verbindung verbessert wird. Die gewünschte Verbindung kann jedes von C. *glutamicum* hergestellte Produkt sein, einschließlich der Endprodukte von Biosynthesewegen und Zwischenprodukte natürlich vorkommender Stoffwechselwege sowie Moleküle, die im Metabolismus von *C. glutamicum* nicht natürlich vorkommen, die jedoch von einem erfindungsgemäßen *C. glutamicum-*Stamm produziert werden.

Diese Erfindung wird durch die nachstehenden Beispiele weiter veranschaulicht, die nicht als einschränkend aufgefaßt werden sollen. Die Inhalte sämtlicher, in dieser Patentanmeldung zitierter Literaturstellen, Patentanmeldungen, Patente und veröffentlichter Patentanmeldungen sind hiermit durch Bezugnahme aufgenommen.

### Beispiele

### Beispiel 1: Präparation der gesamten genomischen DNA aus Corynebacterium glutamicum ATCC13032

Eine Kultur von *Corynebacterium glutamicum* (ATCC 13032) wurde über Nacht bei 30°C unter starkem Schütteln in BHI-Medium (Difco) gezüchtet. Die Zellen wurden durch Zentrifugation geerntet, der Überstand wurde verworfen, und die Zellen wurden in 5ml Puffer I (5% des Ursprungsvolumens der Kultur - sämtliche angegebenen Volumina sind für 100 ml Kulturvolumen berechnet) resuspendiert. Zusammensetzung von Puffer I: 140,34 g/l Saccharose, 2,46 g/l MgSO₄ · 7 H₂O, 10 ml/l KH₂PO₄-Lösung (100g/l, mit KOH auf pH-Wert 6,7 eingestellt), 50 ml/l M12-Konzentrat (10 g/l (NH₄)₂SO₄, 1 g/l NaCl, 2 g/l MgSO₄ · 7 H₂O, 0,2 g/l CaCl₂, 0,5 g/l HefeExtrakt (Difco), 10 ml/l Spurenelemente-Mischung (200 mg/l FeSO₄ · H₂O, 10 mg/l ZnSO₄ · 7 H₂O, 3 mg/l MnCl₂ · 4 H₂O, 30 mg/l H₃BO₃, 20 mg/l CoCl₂ · 6 H₂O, 1 mg/l NiCl₂ · 6 H₂O, 3 mg/l Na₂MoO₄ · 2 H₂O, 500 mg/l Komplexbildner (EDTA oder Citronensäure), 100 ml/l Vitamingemisch (0,2 ml/l Biotin, 0,2 mg/l Folsäure, 20 mg/l p-Aminobenzoesäure, 20 mg/l Riboflavin, 40 mg/l Ca-Panthothenat, 140 mg/l Nikotinsäure, 40 mg/l Pyridoxolhydrochlorid, 200 mg/l Myo-Inositol). Lysozym wurde in einer Endkonzentration von 2,5 mg/ml zur Suspension gegeben. Nach etwa 4 Std. Inkubation bei 37°C wurde die Zellwand abgebaut, und die erhaltenen Protoplasten wurden durch Zentrifugation geerntet. Das Pellet wurde einmal mit 5 ml Puffer I und einmal mit 5 ml TE-Puffer (10 mM Tris-HCl, 1 mM EDTA, pH-Wert 8) gewaschen. Das Pellet wurde in 4 ml TE-Puffer resuspendiert, und 0,5 ml SDS-Lösung (10%) und 0,5 ml NaC1-Lösung (5 M) wurden zugegeben. Nach Zugabe von Proteinase K in einer Endkonzentration von 200 ∝g/ml wurde die Suspension etwa 18 Std. bei 37°C inkubiert. Die DNA wurde durch Extraktion mit Phenol, Phenol-Chloroform-Isoamylalkohol und Chloroform-Isoamylalkohol mittels Standard-Verfahren gereinigt. Dann wurde die DNA durch Zugabe von 1/50 Volumen 3 M Natriumacetat und 2 Volumina Ethanol, anschließender Inkubation für 30 min bei -20°C und 30 min Zentrifugation bei 12000 U/min in einer Hochgeschwindigkeitszentrifuge mit einem SS34-Rotor (Sorvall) gefällt. Die DNA wurde in 1 ml TE-Puffer gelöst, der 20 ∝g/ml RNase A enthielt, und für mindestens 3 Std. bei 4°C gegen 1000 ml TE-Puffer dialysiert. Während dieser Zeit wurde der Puffer 3mal ausgetauscht. Zu Aliquots von 0,4 ml der dialysierten DNA-Lösung wurden 0,4 ml 2 M LiCl und 0,8 ml Ethanol zugegeben. Nach 30 min Inkubation bei -20°C wurde die DNA durch Zentrifugation gesammelt (13000 U/min, Biofuge Fresco, Heraeus, Hanau, Deutschland). Das DNA-Pellet wurde in TE-Puffer gelöst. Durch dieses Verfahren hergestellte DNA konnte für alle Zwecke verwendet werden, einschließlich Southern-Blotting oder zur Konstruktion genomischer Banken.

### Beispiel 2: Konstruktion genomischer Corynebacterium glutamicum (ATCC13032)-Banken in Escherichia coli

Ausgehend von DNA, die wie in Beispiel 1 beschrieben hergestellt wurde, wurden gemäß bekannter und gut eingeführter Verfahren (siehe bspw. Sambrook, J. et al. (1989) "Molecular Cloning: A Laboratory Manual". Cold Spring Harbor Laboratory Press oder Ausubel, F.M. et al. (1994) "Current Protocols in Molecular Biology", John Wiley & Sons) Cosmid- und Plasmid-Banken hergestellt.

Es ließ sich jedes Plasmid oder Cosmid einsetzen. Besondere Verwendung fanden die Plasmide pBR322 (Sutcliffe, J.G. (1979) Proc. Natl Acad. Sci. USA, 75:3737-3741); pACYC177 (Change & Cohen (1978) J. Bacteriol. 134:1141-1156); Plasmide der pBS-Reihe (pBSSK+, pBSSK- und andere; Stratagene, LaJolla, USA) oder Cosmide, wie SuperCos1 (Stratagene, LaJolla, USA) oder Lorist6 (Gibson, T.J. Rosenthal, A., und Waterson, R.H. (1987) Gene 53: 283-286.

### Beispiel 3: DNA-Sequenzierung und Computer-Funktionsanalyse

Genomische Banken, wie in Beispiel 2 beschrieben, wurden zur DNA-Sequenzierung gemäß Standard-Verfahren, insbesondere dem Kettenabbruchverfahren mit ABI377-Sequenziermaschinen (s. z.B. Fleischman, R.D. et al. (1995) "Whole-genome Random Sequencing and Assembly of Haemophilus Influenzae Rd., Science 269:496-512) verwendet. Die Sequenzierprimer mit den folgenden Nukleotidsequenzen wurden verwendet: 5'-GGAAACAGTATGACCATG-3' oder 5'-GTAAAACGACGGCCAGT-3'.

### Beispiel 4: In-vivo-Mutagenese

In vivo-Mutagenese von *Corynebacterium glutamicum* kann durchgeführt werden, indem eine Plasmid- (oder andere Vektor-) DNA durch *E.coli* oder andere Mikroorganismen (z.B. Bacillus spp. oder Hefen, wie *Saccharomyces cerevisiae)* geschleust wird, die die Integrität ihrer genetischen Information nicht aufrechterhalten können. Übliche Mutatorstämme weisen Mutationen in den Genen für das DNA-Reparatursystem auf (z.B., mutHLS, mutD, mutT, usw., zum Vergleich siehe Rupp, W.D. (1996) DNA repair mechanisms, in: *Escherichia coli* and *Salmonella,* S. 2277-2294, ASM: Washington). Diese Stämme sind dem Fachmann bekannt. Die Verwendung dieser Stämme ist bspw. in Greener, A. und Callahan, M. (1994) Strategies 7:32-34 veranschaulicht.

### Beispiel 5: DNA-Transfer zwischen Escherichia coli und Corynebacterium glutamicum

Mehrere *Corynebacterium-* und *Brevibacterium*-Arten enthalten endogene Plasmide (wie bspw. pHM1519 oder pBL1) die autonom replizieren (für einen Überblick siehe bspw. Martin, J.F. et al. (1987) Biotechnology 5:137-146). Shuttle-Vektoren für *Escherichia coli* und *Corynebacterium glutamicum* lassen sich leicht mittels Standard-Vektoren für E. *coli* konstruieren (Sambrook, J. et al., (1989), "Molecular Cloning: A Laboratory Manual", Cold Spring Harbor Laboratory Press oder Ausubel, F.M. et al. (1994) "Current Protocols in Molecular Biology", John Wiley & Sons), denen ein Replikationsursprung für und ein geeigneter Marker aus *Corynebacterium glutamicum* beigegeben wird. Solche Replikationsursprünge werden vorzugsweise von endogenen Plasmiden entnommen, die aus *Corynebacterium-* und *Brevibactertium*-Arten isoliert worden sind. Besondere Verwendung als Transformationsmarker für diese Arten sind Gene für Kanamycin-Resistenz (wie solche, die vom Tn5- oder Tn-903-Transposon stammen) oder für Chloramphenicol (Winnacker, E.L. (1987) "From Genes to Clones - Introduction to Gene Technology, VCH, Weinheim). Es gibt zahlreiche Beispiele in der Literatur für die Herstellung einer großen Vielzahl von Shuttle-Vektoren, die in *E. coli* und *C. glutamicum* replizieren und für verschiedene Zwecke verwendet werden können, einschließlich Gen-Überexpression (siehe bspw. Yoshihama, M. et al. (1985) J. Bacteriol. 162:591-597, Martin, J.F. et al., (1987) Biotechnology, 5:137-146 und Eikmanns, B.J. et al. (1992) Gene 102:93-98).

Mittels Standard-Verfahren ist es möglich, ein Gen von Interesse in einen der vorstehend beschriebenen Shuttle-Vektoren zu klonieren und solche Hybrid-Vektoren in *Corynebacterium glutamicum-*Stämme einzubringen. Die Transformation von *C. glutamicum* läßt sich durch Protoplastentransformation (Kastsumata, R. et al., (1984) J. Bacteriol. 159:306-311), Elektroporation (Liebl, E. et al., (1989) FEMS Microbiol. Letters, 53:399-303) und in Fällen, bei denen spezielle Vektoren verwendet werden, auch durch Konjugation erzielen (wie z.B. beschrieben in Schäfer, A., et (1990) J. Bacteriol. 172:1663-1666). Es ist ebenfalls möglich, die Shuttle-Vektoren für C. *glutamicum* auf E. *coli* zu übertragen, indem Plasmid-DNA aus C. *glutamicum* (mittels im Fachgebiet bekannter Standard-Verfahren) präpariert und in E. *coli* transformiert wird. Dieser Transformationsschritt kann mit Standard-Verfahren erfolgen, jedoch wird vorteilhafterweise ein Mcrdefizienter *E. coli*-Stamm verwendet, wie NM522 (Gough & Murray (1983) J. Mol. Biol. 166:1-19).

### Beispiel 6: Bestimmung der Expression des mutanten Proteins

Die Beobachtungen der Aktivität eines mutierten Proteins in einer transformierten Wirtszelle beruhen auf der Tatsache, daß das mutante Protein auf ähnliche Weise und in ähnlicher Menge exprimiert wird wie das Wildtyp-Protein. Ein geeignetes Verfahren zur Bestimmung der Transkriptionsmenge des mutanten Gens (ein Anzeichen für die mRNA-Menge, die für die Translation des Genprodukts verfügbar ist) ist die Durchführung eines Northern-Blots (s. bspw. Ausubel et al., (1988) Current Protocols in Molecular Biology, Wiley: New York), wobei ein Primer, der so ausgestaltet ist, daß er an das Gen von Interesse bindet, mit einer nachweisbaren (gewöhnlich radioaktiven oder chemilumineszierenden) Markierung versehen wird, so daß - wenn die Gesamt-RNA einer Kultur des Organismus extrahiert, auf einem Gel aufgetrennt, auf eine stabile Matrix übertragen und mit dieser Sonde inkubiert wird - die Bindung und die Quantität der Bindung der Sonde das Vorliegen und auch die Menge der mRNA für dieses Gen anzeigt. Diese Information ist ein Hinweis auf das Ausmaß der Transkription des mutanten Gens. Gesamt-Zell-RNA läßt sich durch verschiedene Verfahren aus *Corynebacterium glutamicum* isolieren, die im Fachgebiet bekannt sind, wie in Bormann, E.R. et al., (1992) Mol. Microbiol. 6:317-326 beschrieben.

Zur Bestimmung des Vorliegens oder der relativen Menge an Protein, das von dieser mRNA translatiert wird, können Standard-Techniken, wie Western-Blot, eingesetzt werden (s. bspw. Ausubel et al. (1988) "Current Protocols in Molecular Biology", Wiley, New York). Bei diesem Verfahren werden Gesamt-Zellproteine extrahiert, durch Gelelektrophorese aufgetrennt, auf eine Matrix, wie Nitrocellulose, übertragen und mit einer Sonde, wie einem Antikörper, die an das gewünschte Protein spezifisch bindet, inkubiert,. Diese Sonde ist gewöhnlich mit einer chemilumineszierenden oder colorimetrischen Markierung versehen, die sich leicht nachweisen läßt. Das Vorliegen und die beobachtete Menge an Markierung zeigt das Vorliegen und die Menge des gesuchten Mutantenproteins in der Zelle an.

### Beispiel 7: Wachstum von genetisch verändertem Corynebacterium glutamicum - Medien und Anzuchtbedingungen

Genetisch veränderte *Corynebakterien* werden in synthetischen oder natürlichen Wachstumsmedien gezüchtet. Eine Anzahl unterschiedlicher Wachstumsmedien für Corynebakterian sind bekannt und leicht erhältlich (Lieb et al. (1989) Appl. Microbiol. Biotechnol. 32:205-210; von der Osten et al. (1998) Biotechnology Letters 11:11-16; Patent DE 4 120 867; Liebl (1992) "The Genus Corynebacterium", in: The Procaryotes, Bd. II, Balows, A., et al., Hrsg. Springer-Verlag). Diese Medien bestehen aus einer oder mehreren Kohlenstoffquellen, Stickstoffquellen, anorganischen Salzen, Vitaminen und Spurenelementen. Bevorzugte Kohlenstoffquellen sind Zucker, wie Mono-, Di- oder Polysaccharide. Sehr gute Kohlenstoffquellen sind bspw. Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Alkohole und organische Säuren, wie Methanol, Ethanol, Essigsäure oder Milchsäure. Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie NH₄Cl oder (NH₄)₂SO₄, NH₄OH, Nitrate, Harnstoff, Aminosäuren oder komplexe Stickstoffquellen, wie Maisquellwasser, Sojamehl, Sojaprotein, Hefeextrakt, Fleischextrakt und andere.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen. Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure. Die Medien enthalten üblicherweise auch andere Wachstumsfaktoren, wie Vitamine oder Wachstumsförderer, zu denen bspw. Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) oder anderen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Anzuchtbedingungen werden für jedes Experiment gesondert definiert. Die Temperatur sollte zwischen 15°C und 45°C liegen und kann während des Experimentes konstant gehalten oder verändert werden. Der pH-Wert des Mediums sollte im Bereich von 5 bis 8,5, vorzugsweise um 7,0 liegen, und kann durch Zugabe von Puffern zu den Medien aufrechterhalten werden. Ein beispielhafter Puffer für diesen Zweck ist ein Kaliumphosphatpuffer. Synthetische Puffer, wie MOPS, HEPES; ACES usw., können alternativ oder gleichzeitig verwendet werden. Der Anzucht-pH-Wert läßt sich während der Anzucht auch durch Zugabe von NaOH oder NH₄OH konstant halten. Werden komplexe Medienkomponenten, wie HefeExtrakt, verwendet, sinkt der Bedarf an zusätzlichen Puffern, da viele komplexe Verbindungen eine hohe Pufferkapazität aufweisen. Beim Einsatz eines Fermenters für die Anzucht von Mikroorganismen kann der pH-Wert auch mit gasförmigem Ammoniak reguliert werden.

Die Inkubationsdauer liegt gewöhnlich in einem Bereich von mehreren Stunden bis zu mehreren Tagen. Diese Zeit wird so ausgewählt, daß sich die maximale Menge Produkt in der Brühe ansammelt. Die offenbarten Wachstumsexperimente können in einer Vielzahl von Behältern, wie Mikrotiterplatten, Glasröhrchen, Glaskolben oder Glas- oder Metallfermentern unterschiedlicher Größen durchgeführt werden. Zum Screening einer großen Anzahl von Klonen sollten die Mikroorganismen in Mikrotiterplatten, Glasröhrchen oder Schüttelkolben, entweder mit oder ohne Schikanen, gezüchtet werden. Vorzugsweise werden 100-ml-Schüttelkolben verwendet, die mit 10% (bezogen auf das Volumen) des erforderlichen Wachstumsmediums gefüllt sind. Die Kolben sollten auf einem Kreiselschüttler (Amplitude 25 mm) mit einer Geschwindigkeit im Bereich von 100-300 U/min geschüttelt werden. Verdampfungsverluste können durch Aufrechterhalten einer feuchten Atmosphäre verringert werden; alternativ sollte für die Verdampfungsverluste eine mathematische Korrektur durchgeführt werden.

Werden genetisch modifizierte Klone untersucht, sollte auch ein unmodifizierter Kontrollklon oder ein Kontrollklon getestet werden, der das Basisplasmid ohne Insertion enthält. Das Medium wird auf eine OD₆₀₀ von 0,5 - 1,5 angeimpft, wobei Zellen verwendet werden, die auf Agarplatten, wie CM-Platten (10 g/l Glucose, 2,5 g/l NaCl, 2 g/l Harnstoff, 10 g/l Polypepton, 5 g/l Hefeextrakt, 5 g/l Fleischextrakt, 22 g/l Agar pH-Wert 6,8 mit 2 M NaOH), die bei 30°C inkubiert worden sind, gezüchtet wurden. Das Animpfen der Medien erfolgt entweder durch Einbringen einer Kochsalzlösung von *C. glutamicum*-Zellen von CM-Platten oder durch Zugabe einer flüssigen Vorkultur dieses Bakteriums.

### Beispiel 8: In-vitro-Analyse der Funktion mutanter Proteine

Die Bestimmung der Aktivitäten und kinetischen Parameter von Enzymen ist im Fachgebiet gut bekannt. Experimente zur Bestimmung der Aktivität eines bestimmten veränderten Enzyms müssen an die spezifische Aktivität des Wildtypenzyms angepaßt werden, was innerhalb der Fähigkeiten des Fachmann liegt. Überblicke über Enzyme im allgemeinen sowie spezifische Einzelheiten, die die Struktur, Kinetiken, Prinzipien, Verfahren, Anwendungen und Beispiele zur Bestimmung vieler Enzymaktivitäten betreffen, können bspw. in den nachstehenden Literaturstellen gefunden werden: Dixon, M., und Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Hrsg. (1983) The Enzymes, 3. Aufl., Academic Press, New York; Bisswanger, H. (1994) Enzymkinetik, 2. Aufl. VCH, Weinheim (ISBN 3527300325); Bergmeyer, H.U., Bergmeyer, J., Graßl, M. Hrsg. (1983-1986) Methods of Enzymatic Analysis, 3. Aufl. Bd. I-XII, Verlag Chemie: Weinheim; und Ullmann's Encyclopedia of Industrial Chemistry (1987) Bd. A9, "Enzymes", VCH, Weinheim, S. 352-363.

Die Aktivität von Proteinen, die an DNA binden, kann durch viele gut eingeführte Verfahren gemessen werden, wie DNA-Banden-Shift-Assays (die auch als Gelretardations-Assays bezeichnet werden). Die Wirkung dieser Proteine auf die Expression anderer Moleküle kann mit Reportergen-Assays (wie in Kolmar, H. et al., (1995) EMBO J. 14:3895-3904 und den darin zitierten Literaturstellen beschrieben) gemessen werden. Reportergen-Testsysteme sind wohlbekannt und für Anwendungen in pro- und eukaryotischen Zellen etabliert, wobei Enzyme, wie beta-Galactosidase, Grün-Fluoreszenz-Protein und mehrere andere verwendet werden.

Die Bestimmung der Aktivität von Membran-Transportproteinen kann gemäß Techniken, wie sie in Gennis, R.B. (1989) "Pores, Channels and Transporters", in Biomembranes, Molecular Structure and Function, Springer: Heidelberg, S. 85-137; 199-234; und 270-322 beschrieben sind, erfolgen.

### Beispiel 9: Analyse des Einflusses von mutiertem Protein auf die Produktion des gewünschten Produktes

Die Wirkung der genetischen Modifikation in C. *glutamicum* auf die Produktion einer gewünschten Verbindung (wie einer Aminosäure) kann bestimmt werden, indem die modifizierten Mikroorganismen unter geeigneten Bedingungen (wie den vorstehend beschriebenen) gezüchtet werden und das Medium und/oder die zellulären Komponenten bezüglich der erhöhten Produktion des gewünschten Produktes (d.h. einer Aminosäure) untersucht wird/werden. Solche Analysetechniken sind dem Fachmann wohlbekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (s. bspw. Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A. et al. (1988) Bioseparations: downstream processing for Biotechnology, John Wiley and Sons; Kennedy, J.F. und Cabral, J.M.S. (1992) Recovery processes for biological Materials, John Wiley and Sons; Shaeiwitz, J.A. und Henry, J.D. (1988) Biochemical Separations, in Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Zusätzlich zur Messung des Fermentationsendproduktes ist es ebenfalls möglich, andere Komponenten der Stoffwechselwege zu analysieren, die zur Produktion der gewünschten Verbindung verwendet werden, wie Zwischen- und Nebenprodukte, um die Gesamt-Effizienz der Produktion der Verbindung zu bestimmen. Die Analyseverfahren umfassen Messungen der Nährstoffmengen im Medium (bspw. Zucker, Kohlenwasserstoffe, Stickstoffquellen, Phosphat und andere Ionen), Messungen der Biomassezusammensetzung und des Wachstums, Analyse der Produktion gemeinsamer Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsg. IRL Press, S. 103-129; 131-163 und 165-192 (ISBN: 0199635773) und den darin angegebenen Literaturstellen beschrieben.

### Beispiel 10: Reinigung des gewünschten Produktes aus C. glutamicum-Kultur

Die Gewinnung des gewünschten Produktes aus *C. glutamicum*-Zellen oder aus dem Überstand der vorstehend beschriebenen Kultur kann durch verschiedene, im Fachgebiet bekannte Verfahren erfolgen. Wird das gewünschte Produkt von den Zellen nicht sezerniert, können die Zellen aus der Kultur durch langsame Zentrifugation geerntet werden, die Zellen können durch Standard-Techniken, wie mechanische Kraft oder Ultraschallbehandlung, lysiert werden. Die Zelltrümmer werden durch Zentrifugation entfernt, und die Überstandsfraktion, die die löslichen Proteine enthält, wird zur weiteren Reinigung der gewünschten Verbindung erhalten. Wird das Produkt von den *C. glutamicum*-Zellen sezerniert, werden die Zellen durch langsame Zentrifugation aus der Kultur entfernt, und die Überstandsfraktion wird zur weiteren Reinigung behalten.

Die Überstandsfraktion aus beiden Reinigungsverfahren wird einer Chromatographie mit einem geeigneten Harz unterworfen, wobei das gewünschte Molekül entweder auf dem Chromatographieharz zurückgehalten wird, viele Verunreinigungen in der Probe jedoch nicht, oder die Verunreinigungen auf dem Harz zurückbleiben, die Probe hingegen nicht. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung für ein bestimmtes zu reinigendes Molekül bewandert. Das gereinigte Produkt kann durch Filtration oder Ultrafiltration konzentriert und bei einer Temperatur aufbewahrt werden, bei der die Stabilität des Produktes maximal ist.

Im Fachgebiet sind viele Reinigungsverfahren bekannt, und das vorhergehende Reinigungsverfahren soll nicht einschränkend sein. Diese Reinigungstechniken sind bspw. beschrieben in Bailey, J.E. & Ollis, D.F. Biochemical Engineering Fundamentals, McGraw-Hill: New York (1986).

Die Identität und Reinheit der isolierten Verbindungen kann durch Techniken des Standes der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

### Äquivalente

Der Fachmann erkennt oder kann - indem er lediglich Routineverfahren verwendet - viele Äquivalente der erfindungsgemäßen spezifischen Ausführungsformen feststellen. Diese Äquivalente sollen von den nachstehenden Patentansprüchen umfaßt sein.

### Die Angaben in Tabelle 1 sind folgendermassen zu verstehen:

In Spalte 1"DNA-ID" bezieht sich die jeweilige Zahl auf die SEQ ID NO des anhängenden Sequenzprotokolls. Eine "5" in der Spalte "DNA-ID" bedeutet demzufolge ein Verweis auf SEQ ID NO:5.

In Spalte 2"AS-ID" bezieht sich die jeweilige Zahl auf die SEQ ID NO des anhängenden Sequenzprotokolls. Eine "6" in der Spalte "AS-ID" bedeutet demzufolge ein Verweis auf SEQ ID NO:6.

In Spalte 3"Identifikation" wird eine eindeutige interne Bezeichnung für jede Sequenz aufgeführt.

In Spalte 4 "AS-POS" bezieht sich die jeweilige Zahl auf die Aminosäureposition der Polypeptidsequenz "AS-ID" in der gleichen Zeile. Eine "26" in der Spalte "AS-POS" bedeutet demzufolge die Aminosäureposition 26 der entsprechend angegebenen Polypeptidsequenz. Die Zählung der Position beginnt N-Terminal bei +1.

In Spalte 5 "AS-Wildtyp" bezeichnet der jeweilige Buchstabe die Aminosäure - dargestellt im Ein-Buchstaben-Code- an der in Spalte 4 angegebenen Position beim entsprechenden Wildtyp-Stamm.

In Spalte 6 "AS-Mutante" bezeichnet der jeweilige Buchstabe die Aminosäure - dargestellt im Ein-Buchstaben-Code- an der in Spalte 4 angegebenen Position beim entsprechenden Mutanten-Stamm.

In Spalte 7"Funktion" wird die physiologische Funktion der entsprechenden Polypeptidsequenz aufgeführt.

### Ein-Buchstaben-Code der proteinogenen Aminosäuren:

- A: Alanin
- C: Cystein
- D: Aspartat
- E: Glutamat
- F: Phenylalanin
- G: Glycin
- H: His
- I: Isoleucin
- K: Lysin
- L: Leucin
- M: Methionin
- N: Asparagin
- P: Prolin
- Q: Glutamin
- R: Arginin
- S: Serin
- T: Threonin
- V: Valin
- W: Tryptophan
- Y: Tyrosin

**Tabelle 1 Gene die für genetische Stabilitäts-, Genexpressions- und Faltungsproteine codieren**

| **DNA ID:** | **AS ID:** | **Identifikation:** | **AS Pos:** | **AS Wildtyp** | **AS Mutante** | **Funktion:** |
|---|---|---|---|---|---|---|
| 1 | 2 | RXA00019 | 337 | P | S | SINGLE-STRANDED-DNA-SPECIFIC EXONUCLEASE RECJ (EC 3.1.-.-) |
| | | | 405 | T | I | SINGLE-STRANDED-DNA-SPECIFIC EXONUCLEASE RECJ (EC 3.1.-.-) |
| | | | 504 | P | S | SINGLE-STRANDED-DNA-SPECIFIC EXONUCLEASE RECJ (EC 3.1.-.-) |
| 3 | 4 | RXA00061 | 754 | S | N | DNA POLYMERASE I (EC 2.7.7.7) |
| 5 | 6 | RXA00209 | 414 | V | A | GLUTAMYL-TRNA(GLN) AMIDOTRANSFERASE SUBUNIT A (EC 6.3.5.-) |
| | | | 454 | L | F | GLUTAMYL-TRNA(GLN) AMIDOTRANSFERASE SUBUNIT A (EC 6.3.5.-) |
| 7 | 8 | RXA00211 | 44 | V | I | GLUTAMYL-TRNA(GLN) AMIDOTRANSFERASE SUBUNIT B (EC 6.3.5.-) |
| 9 | 10 | RXA00314 | 319 | E | K | CYSTEINYL-TRNA SYNTHETASE (EC 6.1.1.16) |
| 11 | 12 | RXA00458 | 170 | L | F | GLUTAMYL-TRNA SYNTHETASE (EC 6.1.1.17) |
| 13 | 14 | RXA00493 | 363 | A | T | 60 KD CHAPERONIN GROEL |
| 15 | 16 | RXA00588 | 23 | A | V | TRANSCRIPTION ELONGATION FACTOR GREA |
| 17 | 18 | RXA00669 | 68 | A | T | TRNA PSEUDOURIDINE SYNTHASE A (EC 4.2.1.70) |
| 19 | 20 | RXA01061 | 686 | P | S | LEUCYL-TRNA SYNTHETASE (EC 6.1.1.4) |
| 21 | 22 | RXA01277 | 704 | G | S | PROLYL ENDOPEPTIDASE (EC 3.4.21.26) |
| 23 | 24 | RXA01278 | 543 | T | I | Protein Translation Elongation Factor G (EF-G) |
| 25 | 26 | RXA01284 | 164 | D | N | Baderial Protein Translation Elongation Factor Tu (EF-TU) |
| | | | 362 | S | F | Bacterial Protein Translation Elongation Factor Tu (EF-TU) |
| 27 | 28 | RXA01344 | 5 | P | L | DNA-DIRECTED RNA POLYMERASE BETA CHAIN (EC 2.7.7.6) |
| | | | 429 | D | V | DNA-DIRECTED RNA POLYMERASE BETA CHAIN (EC 2.7.7.6) |
| 29 | 30 | RXA01345 | 308 | A | V | DNAK PROTEIN |
| 31 | 32 | RXA01404 | 108 | T | I | TRANSCRIPTIONAL REPRESSOR |
| 33 | 34 | RXA01431 | 46 | A | T | THIOREDOXIN |
| 35 | 36 | RXA01438 | 182 | A | T | FERREDOXIN-NADP REDUCTASE (EC 1.18.1.2) |
| 37 | 38 | RXA01490 | 277 | A | V | TRNA PSEUDOURIDINE SYNTHASE B (EC 4.2.1.70) |
| 39 | 40 | RXA01493 | 32 | A | V | Na⁺ DRIVEN MULTIDRUG EFFLUX PUMP |
| 41 | 42 | RXA01559 | 400 | T | A | PROTEIN TRANSLOCASE SUBUNIT SECD |
| 43 | 44 | RXA01596 | 334 | R | C | DNA REPAIR PROTEIN RECN |
| | | | 493 | G | D | DNA REPAIR PROTEIN RECN |
| 45 | 46 | RXA01651 | 7 | S | F | TRANSPOSASE |
| | | | 33 | L | F | TRANSPOSASE |
| 47 | 48 | RXA01710 | 69 | P | S | TRANSCRIPTIONAL REGULATOR |
| 49 | 50 | RXA01852 | 120 | P | S | HISTIDYL-TRNA SYNTHETASE (EC 6.1.1.21) |
| 51 | 52 | RXA01913 | 61 | L | F | Protein Translation Elongation Factor Ts (EF-Ts) |
| 53 | 54 | RXA02145 | 321 | P | L | MENAQUINOL-CYTOCHROME C REDUCTASE CYTOCHROME B SUBUNIT |
| 55 | 56 | RXA02236 | 87 | L | F | integration host factor |
| 57 | 58 | RXA02267 | 65 | A | T | DNA (CYTOSINE-5)-METHYLTRANSFERASE (EC 2.1.1.37) |
| 59 | 60 | RXA02280 | 502 | A | V | HEAT SHOCK PROTEIN HTPG |
| 61 | 62 | RXA02388 | 401 | E | K | COME OPERON PROTEIN 3 |
| | | | 451 | V | M | COME OPERON PROTEIN 3 |
| 63 | 64 | RXA02416 | 484 | G | D | EXCINUCLEASE ABC SUBUNIT A |
| 65 | 66 | RXA02418 | 45 | V | I | Bacterial Protein Translation Initiation Factor 3 ( IF-3) |
| 67 | 68 | RXA02429 | 670 | M | I | PROTEIN TRANSLOCASE SUBUNIT SECA |
| 69 | 70 | RXA02431 | 73 | A | V | DNA POLYMERASE IV |
| 71 | 72 | RXA02445 | 17 | G | E | ATP-DEPENDENT DNA HELICASE RECG |
| 73 | 74 | RXA02476 | 167 | S | F | A/G-SPECIFIC ADENINE GLYCOSYLASE (EC 3.2.2.-) |
| 75 | 76 | RXA02726 | 286 | A | V | ISOLEUCYL-TRNA SYNTHETASE (EC 6.1.1.5) |
| 77 | 78 | RXA02731 | 374 | E | K | EXCINUCLEASE ABC SUBUNIT B |
| | | | 398 | M | L | EXCINUCLEASE ABC SUBUNIT B |
| | | | 410 | R | L | EXCINUCLEASE ABC SUBUNIT B |
| 79 | 80 | RXA02736 | 312 | S | F | PUTATIVE OXPPCYCLE PROTEIN OPCA |
| 81 | 82 | RXA02742 | 179 | G | S | DNA/RNA HELICASE (DEAD/DEAH BOX FAMILY) |
| 83 | 84 | RXA02748 | 100 | P | S | SIGNAL RECOGNITION PARTICLE, SUBUNIT FFH/SRP54 |
| | | | 164 | G | D | SIGNAL RECOGNITION PARTICLE, SUBUNIT FFH/SRP54 |
| 85 | 86 | RXA03070 | 249 | A | V | TRANSPOSASE |
| 87 | 88 | RXA03098 | 164 | S | N | DNA alkylation repair enzyme |
| 89 | 90 | RXA03206 | 98 | G | D | D-Tyr-tRNATyr deacylase |
| 91 | 92 | RXA03260 | 56 | S | F | TRANSPOSASE |
| 93 | 94 | RXA03394 | 11 | S | F | METHIONYL-TRNA SYNTHETASE (EC 6.1.1.10) |
| 95 | 96 | RXA03674 | 342 | V | I | ATP-DEPENDENT HELICASE HEPA |
| 97 | 98 | RXA03793 | 414 | A | V | RNA POLYMERASE SIGMA FACTOR RPOD |
| 99 | 100 | RXA06048 | 3 | L | F | PS1 PROTEIN PRECURSOR |
| | | | 4 | L | P | PS1 PROTEIN PRECURSOR |
| | | | 5 | T | S | PS1 PROTEIN PRECURSOR |
| | | | 9 | A | T | PS1 PROTEIN PRECURSOR |
| | | | 26 | I | V | PS1 PROTEIN PRECURSOR |
| | | | 31 | S | T | PS1 PROTEIN PRECURSOR |
| | | | 66 | S | N | PS1 PROTEIN PRECURSOR |
| | | | 158 | N | D | PS1 PROTEIN PRECURSOR |
| 101 | 102 | RXA07005 | 339 | P | S | PROBABLE ATP-DEPENDENT HELICASE LHR (EC 3.6.1.-) |
| 103 | 104 | RXA07006 | 239 | P | L | EXODEOXYRIBONUCLEASE VII LARGE SUBUNIT (EC 3.1.11.6) |

## Patentansprüche

1. Isoliertes Nukleinsäuremolekül codierend für ein Polypeptid der in Tabelle 1 in Bezug genommenen Aminosäuresequenz ID 80, wobei das Nukleinsäuremolekül in der angegebenenen Aminosäureposition eine andere proteinogene Aminosäure codiert als Serin.

2. Isoliertes Nukleinsäuremolekül nach Anspruch 1, wobei das Nukleinsäuremolekül in der angegebenenen Aminosäureposition 312 für Phenylalanin codiert.

3. Ein Vektor, der wenigstens eine Nukleinsäuresequenz nach Anspruch 1 enthält.

4. Eine Wirtszelle, die mit wenigstens einem Vektor nach Anspruch 3 transfiziert ist.

5. Eine Wirtszelle nach Anspruch 4, wobei die Expression des besagten Nukleinsäuremoleküls zur Modulation der Produktion einer Feinchemikalie aus besagter Zelle führt.

6. Verfahren zur Herstellung einer Feinchemikalie welches die Kultivierung einer Zelle beinhaltet, die mit wenigstens einen Vektor nach Anspruch 3 transfiziert worden ist, so dass die Feinchemikalie produziert wird.

7. Verfahren nach Anspruch 6, **dadurch gekennzeichnet, daß** die Feinchemikalie eine Aminosäure ist.

8. Verfahren nach Anspruch 7, **dadurch gekennzeichnet, dass** die Aminosäure Lysin ist.
